# EUROPEAN PATENT SPECIFICATION

(11) **EP 1 908 772 B1**
(45) Date of publication and mention of the grant of the patent: **23.11.2011**
(21) Application number: 08100586.0
(22) Date of filing: 10.12.1998
(51) Int. Cl.: C12N 15/31, C07K 14/195, A61K 38/00, A61K 38/16, A61K 39/00

(54) **Porphorymonas gingivalis polypeptides and polynucleotides**
Polypeptide und Nukleinsäuren von Phorphorymonas gingivalis
Polypeptides et polynucleotides de Phorphorymonas gingivalis

(30) Priority: 10.12.1997 AU PP083997; 31.12.1997 AU PP118297; 30.01.1998 AU PP154698; 10.03.1998 AU PP226498; 09.04.1998 AU PP291198; 23.04.1998 AU PP312898; 05.05.1998 AU PP333898; 22.05.1998 AU PP365498; 29.07.1998 AU PP491798; 30.07.1998 AU PP496398; 04.08.1998 AU PP502898
(43) Date of publication of application: 09.04.2008
(62) Divisional of application: 06008722.8
(73) Proprietor: CSL Limited, Parkville, VIC 3052 (AU); The University of Melbourne, Parkville, VIC 3052 (AU)
(72) Inventor: Ross, Bruce Carter, Coburg, Victoria VIC 3058 (AU); Barr, Ian George, Templestowe, Victoria VIC 3106 (AU); Patterson, Michelle Anne, Laverton, Victoria VIC 3028 (AU); Agius, Catherine Therese, Box Hill South, Victoria VIC 3128 (AU); Rothel, Linda Joy, Glen Huntly, Victoria VIC 3163 (AU); Margetts, Mai Brigid, Moonee Ponds, Victoria VIC 3039 (AU); Hocking, Dianna Margaret, Flemington, Victoria VIC 3031 (AU); Webb, Elizabeth Ann, Eltham, Victoria VIC 3422 (AU)
(74) Representative: Lee, Nicholas John

(56) References cited:
- EP-A- 0 726 314
- WO-A-96/17936
- WO-A-97/16542
- WO-A-98/49192
- US-A- 5 523 390
- CHEN H A ET AL: "Immunodominant antigens of Porphyromonas gingivalis in patients with rapidly progressive periodontitis" ORAL MICROBIOLOGY AND IMMUNOLOGY, vol. 10, no. 4, 1995, pages 193-201, XP008037635 ISSN: 0902-0055
- NAKAYAMA K ET AL: "CONSTRUCTION AND CHARACTERIZATION OF ARGININE-SPECIFIC CYSTEINE PROTEINASE (ARG-GINGIPAIN)-DEFICIENT MUTANTS OF PORPHYROMONAS GINGIVALIS" JOURNAL OF BIOLOGICAL CHEMISTRY, AL, vol. 270, no. 40, 6 October 1995 (1995-10-06), pages 23619-23626, XP001150726 ISSN: 0021-9258
- MILLAR D J ET AL: "PRODUCTION AND CHARACTERISATION OF MONOCLONAL ANTIBODIES TO THE PRINCIPLE SONICATE ANTIGENS OF PORPHYROMONAS GINGIVALIS W50" FEMS IMMUNOLOGY AND MEDICAL MICROBIOLOGY, ELSEVIER SCIENCE B.V., AMSTERDAM, NL, vol. 7, no. 3, 1 January 1993 (1993-01-01), pages 211-222, XP008075003 ISSN: 0928-8244
- SLAKESKI NADA ET AL: "Characterization of a second cell-associated Arg-specific cysteine proteinase of Porphyromonas gingivalis and identification of an adhesin-binding motif involved in association of the prtR and prtK proteinases and adhesins into large complexes" MICROBIOLOGY, SOCIETY FOR GENERAL MICROBIOLOGY, READING, GB, vol. 144, no. 6, 1 June 1998 (1998-06-01), pages 1583-1592, XP002215376 ISSN: 1350-0872
- DATABASE UniProt [Online] 1 November 1996 (1996-11-01), "SubName: Full=Surface layer protein B; Methanosarcina mazei" XP002500360 retrieved from EBI accession no. UNIPROT:Q50244 Database accession no. Q50244
- SETTLE JR S H ET AL: "The silver gene of Drosophila melanogaster encodes multiple carboxypeptidases similar to mammalian prohormone-processing enzymes" PROCEEDINGS OF THE NATIONAL ACADEMY OF SCIENCES OF THE UNITED STATES OF AMERICA 19951010 US, vol. 92, no. 21, 10 October 1995 (1995-10-10), pages 9470-9474, XP002500358 ISSN: 0027-8424
- TAN F ET AL: "Molecular cloning and sequencing of the cDNA for human membrane-bound carboxypeptidase M. Comparison with carboxypeptidases A, B, H, and N" JOURNAL OF BIOLOGICAL CHEMISTRY 1989 US, vol. 264, no. 22, 1989, pages 13165-13170, XP002500359 ISSN: 0021-9258
- POTEMPA J ET AL: "Comparative properties of two cysteine proteinases (gingipains R), the products of two related but individual genes of Porphyromonas gingivalis" JOURNAL OF BIOLOGICAL CHEMISTRY 19980821 US, vol. 273, no. 34, 21 August 1998 (1998-08-21), pages 21648-21657, XP002485021 ISSN: 0021-9258
- NELSON K E ET AL: "Complete genome sequence of the oral pathogenic Bacterium porphyromonas gingivalis strain W83" JOURNAL OF BACTERIOLOGY, WASHINGTON, DC, US, vol. 185, no. 18, 1 September 2003 (2003-09-01), pages 5591-5601, XP002320223 ISSN: 0021-9193
- MOLHOJ MICHAEL ET AL: "Leader sequences are not signal peptides.", NATURE BIOTECHNOLOGY DEC 2004 LNKD- PUBMED:15583649, vol. 22, no. 12, December 2004 (2004-12), page 1502, ISSN: 1087-0156
- ROSS B C ET AL: "Identification of vaccine candidate antigens from a genomic analysis of Porphyromonas gingivalis", VACCINE, ELSEVIER LTD, GB, vol. 19, no. 30, 20 July 2001 (2001-07-20) , pages 4135-4142, XP004255129, ISSN: 0264-410X, DOI: DOI:10.1016/S0264-410X(01)00173-6
- VEITH P D ET AL: "Porphyromonas gingivalis RgpA and Kgp proteinases and adhesins are C terminally processed by the carboxypeptidase CPG70", INFECTION AND IMMUNITY 200406 US LNKD- DOI:10.1128/IAI.72.6.3655-3657.2004, vol. 72, no. 6, June 2004 (2004-06), pages 3655-3657, ISSN: 0019-9567
- CHEN Y -Y ET AL: "CPG70 is a novel basic metallocarboxypeptidase with C-terminal polycystic kidney disease domains from Porphyromonas gingivalis", JOURNAL OF BIOLOGICAL CHEMISTRY 20020628 US LNKD- DOI:10.1074/JBC.M200811200, vol. 277, no. 26, 28 June 2002 (2002-06-28), pages 23433-23440, ISSN: 0021-9258
- ANG C -S ET AL: "Application of <16>O/<18>O reverse proteolytic labeling to determine the effect of biofilm culture on the cell envelope proteome of Porphyromonas gingivalis W50", PROTEOMICS 200804 DE LNKD- DOI:10.1002/PMIC.200700557, vol. 8, no. 8, April 2008 (2008-04), pages 1645-1660, ISSN: 1615-9853

(2) INFORMATION FOR SEQ ID NO:1
   (i) SEQUENCE: CHARACTERISTICS:
      (A) LENGTH: 1362 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: PORYPHYROMOHAS GINGIVALIS
   (ix) FEATURE:
      (A) NAME/KEY: misc_feature
      (B) LOCATION 1...1362
   (xi) SEQUENCE DESCRIPTION: SEQ ID NO:1
   TTCTGTGTCA TGGCAAAAGT TATAAAAACA AAAAAAGGCC TTGCACTTAA TCTGAAAGGA     60
   AAACCGCTGC CCGAGATGCT GGCCGAACCG GCCCAAAGTC CTACTTACGC GGTCGTGCCC    120
   GACGATTTTG AAGGTGTTAT CCCCAAGGTG ACGGCTCGTC CGGGGGATAA GGTGCGTGCC     180
   GGCTCAGCAC TGATGCACCA CAAGGCATAT CCGGAGATGA AGTTTACAAG TCCGGTTAGC    240
   GGCGAAGTGA TCGCGGTGAA TCGCGGTGCC AAGCGCAAGG TGTTGAGCAT CGAGGTGAAA    300
   CCGGACGGAC TGAACGAATA CGAGTCATTC CCTGTCGGGG ATCCGTCTGC CCTCTCTGCC    360
   GAACAGATCA AGGAGCTTTT ACTGTCGAGC GGTATGTGGG GTTTTATTAA GCAACGTCCT    420
   TACGACATAG TGGCTACACC GGATATAGCT CCACGCGACA TTTATATTAC TGCCAACTTT    480
   ACTGCACCAT TGGCTCCGGA CTTCGATTTC ATCGTTCGAG GAGAAGAACG CGCCCTGCAG    540
   ACTGCCATCG ATGCCTTGGC CAAACTCACG ACAGGAAAGG TGTATGTGGG CCTGAAGCCG    600
   GGTTCATCTC TGGGCTTGCA CAATGCAGAA ATCGTAGAAG TACACGGACC TCATCCGGCA    660
   GGTAACCTGG GCGTGCTGAT CAATCATACG AAGCCAATCA ATCGGGGCGA AACGGTGTGG    720
   ACGCTCAAGG CTACCGACCT GATCGTGATC GGACGTTTCC TGCTTACGGG CAAAGCCGAT    780
   TTTACCAGAA TGATTGCCAT GACCGGCTCA GACGCTGCAG CTCACGGATA CGTCCGTATT    840
   ATGCCGGGTT GCAATGTCTT TGCTTCCTTC CCCGGCCGAC TGACAATAAA GGAATCTCAC    900
   GAGCGTGTGA TCGATGGCAA TGTGCTGACC GGTAAGAAGC TCTGCGAGAA GGAGCCTTTC    960
   CTGTCAGCCC GGTGTGACCA GATCACGGTG ATCCCCGAAG GCGACGATGT GGACGAACTC   1020
   TTCGGGTGGG CTGCACCCCG TCTCGATCAG TACAGCATGA GCAGAGCTTA TTTCTCTTGG   1080
   TTGCAGGGGA AAAACAAAGA GTACGTACTC GATGCCCGGA TCAAGGGTGG CGAACGTGCT   1140
   ATGATCATGA GCAACGAGTA TGACCGCGTT TTCCCGATGG ACATCTATCC GGAGTATTTG   1200
   CTCAAGGCTA TTATAGCATT CGACATCGAC AAGATGGAGG ACTTAGGCAT ATATGAAGTG   1260
   GCTCCGGAGG ACTTTGCCAC TTGCGAATTT GTGGATACAT CCAAGATCGA GCTGCAGCGT   1320
   ATCGTTCGCG AGGGCTTGGA TATGCTCTAT AAGGAAATGA AT                      1362
(2) INFORMATION FOR SEQ ID NO:37
   (i) SEQUENCE CHARACTERISTICS:
      (A) LENGTH: 2607 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISH: PORTPHYROMONAS GINGIVALIS
   (ix) FEATURE:
      (A) NAME/KEY: misc_feature
      (B) LOCATION 1...2607
   (xi) SEQUENCE DESCRIPTION: SEQ ID NO:37
   TGGCATAGGA ATATTTTTAT CTTTGCGAGT ACATTTAGCC CGAAAAATAT GCTCCCACTG     60
   CCATACCGTT ATGCAAAAAC CGAGCACCTT TTTCTCGCAA AAGGATACTG CAAGAATCCA    120
   ATAACAAACA TAATTATCCT ATTTATGAAG AAAAAGAATT TTTTGCTTCT TGGCATTTTC    180
   GTTGCTTTGC TGACTTTCAT CGGCAGCATG CAGGCACAAC AGGCCAAAGA TTATTTCAAC    240
   TTTGACGAAC GGGGCGAGGC CTACTTCTCA TTCAAAGTGC CTGATAGGGC CGTTCTACAA    300
   GAGCTGGCTC TGATCATGTC CATCGACGAG TTTGACCCCG TAACCAATGA AGCCATTGCC    360
   TATGCCAGCG AAGAGGAGTT CGAGGCATTC CTGCGCTATG GGCTCAAGCC TACATTCTTG    420
   ACTCCTCCAT CCATGCAGCG CGCTGTCGAG ATGTTCGACT ACCGCTCAGG AGAAAAATAC    480
   GAATGGAATG CTTACCCCAC CTATGAAGCC TATATCAGCA TGATGGAAGA GTTCCAAACA    540
   AAGTATCCAT CACTTTGTAC TACTTCCGTC ATTGGCAAGT CCGTAAAGGA TCGTAAACTG    600
   ATGATTTGCA AGCTGACGTC CTCTGCCAAT ACAGGGAAAA AGCCTCGCGT GCTCTATACT    660
   TCTACGATGC ACGGAGACGA AACGACCGGA TATGTGGTAC TGCTCCGACT CATAGACCAT    720
   CTGCTGTCGA ACTACGAATC CGATCCGAGG ATTAAGAACA TTCTGGATAA AACGGAAGTA    780
   TGGATCTGCC CTTTGACCAA TCCGGACGGA GCATACAGAG CCGGAAACCA CACCGTACAA    840
   GGAGCTACTC GCTACAATGC CAACAATGTC GATTTGAACC GTAACTTCAA GGATGATGTA    900
   GCCGGTGATC ACCCCGATGG AAAACCTTGG CAGCCGGAGG CAACTGCATT CATGGATTTG    960
   GAAGGAAACA CCTCTTTCGT GCTCGGTGCC AATATACATG GAGGAACAGA GGTGGTGAAC   1020
   TATCCATGGG ATAATAAAAA AGAAAGACAT GCAGACGATG AGTGGTACAA ACTGATCAGT   1080
   CGCAACTACG CAGCCGCTTG TCAGAGTATT TCCGCCAGCT ACATGACCTC CGAAACCAAT   1140
   TCGGGAATCA TCAACGGTTC AGACTGGTAT GTAATTCGCG GAAGTCGTCA GGACAATGCA   1200
   AATTATTTCC ATCGTCTGCG AGAAATTACC CTTGAAATCA GCAACACGAA GTTGGTGCCG   1260
   GCCTCTCAAC TTCCAAAGTA TTGGAATCTG AACAAAGAAT CTCTGCTTGC TCTGATCGAA   1320
   GAATCCTTAT ACGGCATCCA TGGTACAGTG ACTTCCGCTG CGAACGGACA GCCTCTCAAA   1380
   TGCCAGATCT TGATAGAAAA CCATGACAAG CGCAACTCCG ATGTTTACTC CGATGCTACC   1440
   ACAGGCTACT ACGTACGTCC TATCAAAGCC GGCACTTATA CGGTGAAATA CAAAGCCGAG   1500
   GGTTATCCTG AGGCAACTCG TACCATTACG ATCAAGGACA AAGAAACCGT CATCATGGAC   1560
   ATTGCATTGG GCAACTCGGT TCCTCTGCCT GTACCCGATT TCACAGCTTC TCCTATGACC   1620
   ATCTCAGTAG GCGAAAGCGT CCAATTCCAA GATCAAACGA CAAATAACCC CACGAATTGG   1680
   GAGTGGACGT TCGAAGGCGG ACAGCCTGCC ATGAGTACAG AGCAGAATCC GCTCGTATCC   1740
   TATAGTCATC CCGGTCAGTA CGACGTTACG CTCAAAGTGT GGAATGCAAG TGGTTCCAAC   1800
   ACGATTACGA AAGAAAAATT CATCACTGTC AATGCCGTTA TGCCTGTAGC TGAATTCGTC   1860
   GGTACCCCGA CGGAAATAGA AGAGGGCCAG ACGGTATCTT TCCAAAACCA ATCCACCAAT   1920
   GCCACCAACT ACGTATGGAT ATTCGATGGC GGCACTCCCG CTACCAGTGA AGACGAAAAC   1980
   CCGACTGTGC TTTACAGCAA AGCCGGCCAA TACGATGTCA CGCTCAAGGC GATCAGTGCT   2040
   TCCGGTGAAA CGGTGAAGAC GAAAGAAAAA TACATCACTG TCAAGAAAGC TCCGGTCCCT   2100
   GCTCCGGTAG CCGACTTCGA AGGAACACCT CGAAAAGTAA AGAAAGGCGA GACAGTTACT   2160
   TTCAAAGACT TGTCTACGAA CAATCCGACT TCATGGCTTT GGGTGTTCGA AGGCGGCTCT   2220
   CCTGCCACCA GCACGGAGCA AAACCCGGTG GTCACCTACA ATGAAACAGG CAAGTACGAT   2280
   GTCCAGCTGA CTGCCACCAA CGAGGGCGGA AGCAATGTGA AGAAAGCAGA AGACTACATT   2340
   GAGGTTATCC TCGATGACAG TGTCGAGGAC ATAGTGGCAC AGACGGGTAT CGTCATTCGT   2400
   CCGCAAAACG GAACGAAGCA GATCCTCATA GAAGCCAACG CTGCTATCAA AGCGATCGTT   2460
   CTCTATGACA TCAATGGACG GGTCGTACTC AAAACTACTC CGAATCAGCT CCGCTCGACC   2520
   GTAGATCTTT CCATCCTGCC CGAAGGAATC TACACCATCA ATATCAAAAC GGAAAAATCC   2580
   GCTCGCACGG AAAAGATCCA TATCGGG                                       2607
(2) INFORMATION FOR SEQ ID NO: 45
   (i) SEQUENCE CHARACTERISTICS:
      (A) LENGTH: 690 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: PORYPHYROMONAS GINGIVALIS
   (ix) FEATURE:
      (A) NAME/KEY: misc_feature
      (B) LOCATION 1...690
   (xi) SEQUENCE DESCRIPTION: SEQ ID NO:45
   ACGAATAAAA AAGAAGAGAC AATGAAGAAA TCAAGTGTAG TAGCCTCAGT TTTGGCCGTG     60
   GCTCTCGTGT TCGCCGGTTG CGGACTGAAC AATATGGCAA AAGGCGGCCT TATCGGCGCC    120
   GGAGTAGGAG GTGCCATTGG TGCCGGAGTA GGTAACGTAG CCGGAAATAC GGCTGTCGGT    180
   GCCATCGTCG GTACTGCAGT CGGTGGAGCA GCCGGTGCTC TCATCGGAAA GAAGATGGAC    240
   AAGCAGAAAA AAGAACTGGA GGCCGCAGTA CCCGATGCTA CGATTCAGAC AGTAAATGAC    300
   GGAGAGGCTA TTCTGGTTAC TTTCGATAGC GGTATCCTCT TTGCGACGAA CTCCAGCACT    360
   CTGAGTCCCA ACTCACGCAC TGCGCTGACG AAGTTTGCTG CAAACATGAA CAAAAACCCC    420
   GACACGGATA TTCGTATCGT AGGCCATACG GACAATACCG GCTCCGACAA GATCAACGAT    480
   CCTCTGTCTG AGAGACGTGC AGCCAGCGTA TATTCTTTCC TGAATTCTCA GGGTGTGAGT    540
   ATGTCGCGCA TGGCAGCCGA AGGGCGTGGG AGCCATGAAC CGGTTGCAGA CAATAGCACA    600
   GTTGCCGGAC GTTCGGCCAA CCGCCGTGTG GAGGTTTATA TCTTGCCGAA TGCCAAGATG    660
   ATCGAACAAG CACAGCAAGG TACGCTGAAG                                     690
(2) INFORMATION FOR SEQ ID NO: 46
   (i) SEQUENCE CHARACTERISTICS:
      (A) LENGTH: 1026 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGAHISM/ PORYPHYROMONAS GINGIVALIS
   (ix) FEATURE:
      (A) NAME/KEY: misc_feature
      (B) LOCATION 1...1026
   (xi) SEQUENCE DESCRIPTION: SEQ ID NO:46
   AACAGGAACA GAAATATGTC GAAAAAATCG ATCCTTCTGC TTTGCTGTTC GCTGTGCTTC     60
   ATTTCTGCTA CGAAGGCTGT GACCCCCGTC AGAAATGTGC GCAATAGCCA AGTGAACAGC    120
   AAAGCAAAGA CCGAACGTAC AAAGCCCTCG GACTCTGTAC GGTACATTAG CAACATGATT    180
   GCAGATCGGC TGGAGTTCCG CAACAAGATT TCTTCCGAAA AAGAGGTAAG AAAAGCCGAA    240
   TATGAAAATC GGCTGGCGAT GGAAGCACTC AATTACCCTG CCATAGATTT ATATGGTGAA    300
   GATTCTTGGA GCGAGTATGT AAACCCTTTC GTGGGTGCAG GAACCGATGT CGAAATTCCG    360
   AACTCCTATG ACATTGATTG CTCTTCGTTC GTGATGCCCG TCGAAGATAA GCAGGTCACC    420
   TCTCAATTTG GCTACCGTCG GCGTTTCGGA CGGATGCACT ATGGTATTGA TCTTTCAGTG    480
   AATCGTGGCG ATACGATACG AGCAGCCTTT GACGGGAAAG TTCGTGTACG CAGCTATGAA    540
   GCGCGTGGCT ATGGCTACTA CATAGTCTTG CGCCATCCGA ACGGACTGGA GACTGTGTAC    600
   GGACACATGA GTCGCCAATT GGTAGACGAG AATCAGATCG TTCGAGCAGG ACAACCGATC    660
   GGATTAGGAG GCAGCACGGG TCGAAGCACC GGTCCTCATC TTCACTTCGA GACCCGCTTC    720
   ATGGGTATTC CCATCAATCC GAGTACCATT ATAGACTTCG ATAACGGAGT GCCGCTCCGA    780
   GACATTTACA CATTCAAACG AGGGAGCAAT TCTCGCTATG CAAAAGCCTC TAAGACTTCT    840
   TCTCGCTATG CAAAAAAAGG GAAGAAAGGC AGACAAGCTT CTTCTCCTAT GACCTATAGA    900
   ATCAAAAAAG GCGATACTTT GGAAACAATA GCCAAAAGGC ACGGCACTTC TGTTCAGAAA    960
   CTCTGTGCTA CCAATGGCAT TGGCAAGAGT AAAATTTTGA CTCCGGGCAA AGCCTTGAGG   1020
   ATCAAA                                                              1026
(2) INFORMATION FOR SEQ ID NO:118
   (i) SEQUENCE CHARACTERISTICS:
      (A) LENGTH: 1689 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: PORYPHYROMONAS GINGIVALIS
   (ix) FEATURE:
      (A) NAME/KEY: misc_feature
      (B) LOCATION 1...1689
   (xi) SEQUENCE DESCRIPTION: SEQ ID NO: 118
   CATCATAAAA CATATCAAAC AATGAAAAAG CTTTTACAGG CTAAAGCCTT GATTCTGGCA    60
   TTGGGACTCT TCCAACTGCC CGCAATCGCC CAAACGCAAA TGCAAGCAGA CCGAACAAAC   120
   GGTCAATTTG CAACAGAAGA GATGCAACGA GCATTCCAGG AAACGAATCC CCCTGCAGGT   180
   CCTGTGCGTG CTATCGCTGA GTACGAACGC TCTGCAGCCG TTTTGGTACG CTACCCGTTC   240
   GGTATCCCGA TGGAATTGAT CAAAGAGCTG GCCAAGAACG ACAAGGTGAT TACCATTGTG   300
   GCGAGTGAAA GCCAAAAAAA CACCGTTATA ACCCAGTACA CCCAAAGCGG TGTGAATCTC   360
   TCTAATTGCG ATTTCATCAT TGCGAAAACT GACTCTTACT GGACACGCGA CTATACCGGT   420
   TGGTTCGCAA TGTACGATAC GAACAAAGTA GGTCTCGTGG ACTTTATTTA TAACCGCCCT   480
   CGTCCTAACG ATGATGAATT CCCCAAATAC GAAGCACAAT ATCTGGGCAT CGAGATGTTC   540
   GGGATGAAGC TCAAGCAGAC CGGTGGCAAC TACATGACGG ACGGATATGG ATCCGCTGTG   600
   CAGTCACATA TCGCATATAC GGAGAACTCC TCTCTGTCTC AAGCTCAAGT AAATCAAAAG   660
   ATGAAAGACT ATCTCGGCAT CACACATCAT GATGTGGTAC AAGATCCGAA CGGCGAATAT   720
   ATCAACCATG TGGACTGTTG GGGCAAGTAT TTGGCACCGA ACAAAATCCT CATCAGGAAA   780
   GTGCCTGACA ATCACCCTCA GCACCAAGCC CTGGAAGATA TGGCAGCCTA CTTCGCAGCA   840
   CAGACCTGCG CATGGGGAAC GAAGTACGAG GTATATCGCG CTTTGGCCAC CAATGAACAA   900
   CCGTACACGA ACTCTCTGAT TCTGAACAAC AGGGTATTTG TTCCTGTCAA TGGCCCCGCC   960
   TCCGTGGACA ACGATGCTCT GAACGTCTAT AAGACGGCAA TGCCCGGTTA CGAAATTATA  1020
   GGTGTCAAAG GGGCTTCAGG AACACCTTGG TTAGGAACAG ATGCCCTGCA TTGTCGTACT  1080
   CACGAGGTAG CGGATAAGGG CTATCTCTAT ATCAAGCACT ACCCGATACT GGGCGAACAG  1140
   GCAGGCCCTG ATTATAAGAT CGAAGCAGAT GTCGTCTCAT GCGCCAATGC TACTATCTCG  1200
   CCGGTACAAT GTTACTATCG TATCAATGGT TCCGGTAGCT TTAAGGCTGC TGATATGACG  1260
   ATGGAATCAA CAGGTCACTA TACTTATAGC TTTACAGGTC TTAACAAGAA TGATAAGGTA  1320
   GAATACTATA TCTCTGCCGC TGACAATAGT GGTCGCAAAG AGACTTATCC CTTTATCGGC  1380
   GAACCTGATC CTTTCAAGTT TACGTGTATG AACGAAACCA ATACATGTAC TGTGACCGGA  1440
   GCTCCCAAAG CTCTTCGTGC ATGGTTCAAC GCCGGTCGTT CAGAACTGGC TGTTTCGGTA  1500
   AGTTTGAATA TTGCCGGCAC ATATCGGATA AAGCTTTATA ACACCGCAGG AGAAGAAGTC  1560
   GCTGCAATGA CCAAGGAATT AGTAGCAGGG ACGAGTGTCT TCAGTATGGA TGTGTATTCT  1620
   CAGGCTCCGG GCACATATGT TCTGGTTGTT GAAGGAAATG GAATCCGTGA GACAATGAAA  1680
   ATTCTCAAA                                                          1689
(2) INFORMATION FOR SEQ ID NO:119
   (i) SEQUENCE CHARACTERISTICS:
      (A) LENGTH: 1311 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: PORYPHYROHONAS GINGIVALIS
   (ix) FEATURE:
      (A) NAME/KEY: misc_feature
      (B) LOCATION 1...1311
   (xi) SEQUENCE DESCRIPTION: SEQ ID NO:119
   ACCACAAATA GAAAACCAAA TACTAATATG AAACTTTCAT CTAAGAAAAT CTTAGCAATC   60
   ATTGCATTGC TGACGATGGG ACATGCTGTG CAGGCACAGT TTGTTCCGGC TCCCACCACA  120
   GGGATTCGCA TGTCTGTCAC TACAACCAAG GCCGTAGGCG AAAAAATCGA ATTGTTGGTT  180
   CATTCCATAG AGAAGAAAGG CATCTGGATC GATCTCAATG GGGATGCCAC TTACCAACAA  240
   GGAGAGGAAA TAACCGTATT CGATGAGGCA TACCACGAAT ACACGATCGG GACGCAAACC  300
   CTCACTATCT ATGGTAATAC GACCCGATTG GGCTGTCGAT CTACCGGTGC AACGGCTGTC  360
   GATGTAACGA AAAACCCTAA TCTGACCTAT CTCGCATGCC CGAAAAATAA TCTGAAATCA  420
   TTGGACTTGA CGCAAAACCC AAAGCTGCTG CGAGTTTGGT GCGACTCTAA CGAAATAGAA  480
   AGTTTGGACC TGAGTGGCAA TCCGGCTTTG ATCATCCTCG GCTGTGACAG GAATAAGCTG  540
   ACTGAGCTGA AGACCGATAA CAACCCCAAG TTGGCCTCTC TTTGGTGTTC TGATAATAAC  600
   CTGACGGAGT TGGAACTCAG TGCCAATCCT CGTCTCAATG ATCTTTGGTG CTTCGGTAAT  660
   CGGATCACGA AACTCGATCT GAGTGCCAAT CCTCTATTGG TAACACTTTG GTGCAGTGAC  720
   AATGAGCTTT CGACCTTGGA TCTTTCCAAG AATTCGGACG TTGCTTACCT TTGGTGTTCA  780
   TCGAACAAAC TTACATCCTT GAATCTGTCG GGGGTGAAGG GACTGAGTGT TTTGGTTTGT  840
   CATTCCAATC AGATCGCAGG TGAAGAAATG ACGAAAGTGG TGAATGCTTT GCCCACACTA  900
   TCTCCCGGCG CAGGCGCTCA GAGCAAGTTC GTCGTTGTAG ACCTCAAGGA CACTGATGAG  960
   AAGAATATCT GTACCGTAAA GGATGTGGAA AAAGCTAAAA GTAAGAACTG GCGAGTATTT 1020
   GACTTCAACG GTGATTCTGA CAATATGCTT CCATACGAAG GAAGTCCGAC ATCGAACTTG 1080
   GCAGTAGATG CTCCCACTGT CAGGATATAT CCCAATCCGG TAGGAAGATA TGCGCTCGTC 1140
   GAGATCCCCG AGTCTCTTTT AGGGCAGGAA GCTGCTTTAT ACGATATGAA TGGGGTAAAA 1200
   GTCTATAGTT TCGCGGTAGA GTCTCTTCGT CAGAACATTG ACCTGACACA TCTTCCCGAC 1260
   GGCACTTATT TCTTCCGTCT CGATAACTAT ACCACTAAGC TCATCAAACA G          1311
(2) INFORMATION FOR SEQ ID NO:122
   (i) SEQUENCE CHARACTERISTICS:
      (A) LENGTH: 1353 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISH: PORYPHYROMONAS GINGIVALIS
   (ix) FEATURE:
      (A) NAME/KEY: misc_feature
      (B) LOCATION 1...1353
   (xi) SEQUENCE DESCRIPTION: SEQ ID NO: 122
   ATGGCAAAAG TTATAAAAAC AAAAAAAGGC CTTGCACTTA ATCTGAAAGG AAAACCGCTG     60
   CCCGAGATGC TGGCCGAACC GGCCCAAAGT CCTACTTACG CGGTCGTGCC CGACGATTTT    120
   GAAGGTGTTA TCCCCAAGGT GACGGCTCGT CCGGGGGATA AGGTGCGTGC CGGCTCAGCA    180
   CTGATGCACC ACAAGGCATA TCCGGAGATG AAGTTTACAA GTCCGGTTAG CGGCGAAGTG    240
   ATCGCGGTGA ATCGCGGTGC CAAGCGCAAG GTGTTGAGCA TCGAGGTGAA ACCGGACGGA    300
   CTGAACGAAT ACGAGTCATT CCCTGTCGGG GATCCGTCTG CCCTCTCTGC CGAACAGATC    360
   AAGGAGCTTT TACTGTCGAG CGGTATGTGG GGTTTTATTA AGCAACGTCC TTACGACATA    420
   GTGGCTACAC CGGATATAGC TCCACGCGAC ATTTATATTA CTGCCAACTT TACTGCACCA    480
   TTGGCTCCGG ACTTCGATTT CATCGTTCGA GGAGAAGAAC GCGCCCTGCA GACTGCCATC    540
   GATGCCTTGG CCAAACTCAC GACAGGAAAG GTGTATGTGG GCCTGAAGCC GGGTTCATCT    600
   CTGGGCTTGC ACAATGCAGA AATCGTAGAA GTACACGGAC CTCATCCGGC AGGTAACGTG    660
   GGCGTGCTGA TCAATCATAC GAAGCCAATC AATCGGGGCG AAACGGTGTG GACGCTCAAG    720
   GCTACCGACC TGATCGTGAT CGGACGTTTC CTGCTTACGG GCAAAGCCGA TTTTACCAGA    780
   ATGATTGCCA TGACCGGCTC AGACGCTGCA GCTCACGGAT ACGTCCGTAT TATGCCGGGT    840
   TGCAATGTCT TTGCTTCCTT CCCCGGCCGA CTGACAATAA AGGAATCTCA CGAGCGTGTG    900
   ATCGATGGCA ATGTGCTGAC CGGTAAGAAG CTCTGCGAGA AGGAGCCTTT CCTGTCAGCC    960
   CGGTGTGACC AGATCACGGT GATCCCCGAA GGCGACGATG TGGACGAACT CTTCGGGTGG   1020
   GCTGCACCCC GTCTCGATCA GTACAGCATG AGCAGAGCTT ATTTCTCTTG GTTGCAGGGG   1080
   AAAAACAAAG AGTACGTACT CGATGCCCGG ATCAAGGGTG GCGAACGTGC TATGATCATG   1140
   AGCAACGAGT ATGACCGCGT TTTCCCGATG GACATCTATC CGGAGTATTT GCTCAAGGCT   1200
   ATTATAGCAT TCGACATCGA CAAGATGGAG GACTTAGGCA TATATGAAGT GGCTCCGGAG   1260
   GACTTTGCCA CTTGCGAATT TGTGGATACA TCCAAGATCG AGCTGCAGCG TATCGTTCGC   1320
   GAGGGCTTGG ATATGCTCTA TAAGGAAATG AAT                                1353
(2) INFORMATION FOR SEQ ID NO: 162
   (i) SEQUENCE CHARACTERISTICS:
      (A) LENGTH: 2463 base pairs (8) TYPE: nucleic acid (C) STRANDEDNESS: double (D) TOPOLOGY: circular (ii) HOLECULE TYPE: DNA (genomic) (iii) HYPOTHETICAL: NO (iv) ANTI-SENSE: NO
   (vi) ORIGINAL SOURCE:
      (A) ORGANISH: PORYPHYROMONAS GUIGIVALIS
   (ix) FEATURE:
      (A) NAME/KEY: misc_feature
      (B) LOCATION 1...2463
   (xi) SEQUENCE DESCRIPTION: SEQ ID NO: 162
   ATGAAGAAAA AGAATTTTTT GCTTCTTGGC ATTTTCGTTG CTTTGCTGAC TTTCATCGGC     60
   AGCATGCAGG CACAACAGGC CAAAGATTAT TTCAACTTTG ACGAACGGGG CGAGGCCTAC    120
   TTCTCATTCA AAGTGCCTGA TAGGGCCGTT CTACAAGAGC TGGCTCTGAT CATGTCCATC    180
   GACGAGTTTG ACCCCGTAAC CAATGAAGCC ATTGCCTATG CCAGCGAAGA GGAGTTCGAG    240
   GCATTCCTGC GCTATGGGCT CAAGCCTACA TTCTTGACTC CTCCATCCAT GCAGCGCGCT    300
   GTCGAGATGT TCGACTACCG CTCAGGAGAA AAATACGAAT GGAATGCTTA CCCCACCTAT    360
   GAAGCCTATA TCAGCATGAT GGAAGAGTTC CAAACAAAGT ATCCATCACT TTGTACTACT    420
   TCCGTCATTG GCAAGTCCGT AAAGGATCGT AAACTGATGA TTTGCAAGCT GACGTCCTCT    480
   GCCAATACAG GGAAAAAGCC TCGCGTGCTC TATACTTCTA CGATGCACGG AGACGAAACG    540
   ACCGGATATG TGGTACTGCT CCGACTCATA GACCATCTGC TGTCGAACTA CGAATCCGAT    600
   CCGAGGATTA AGAACATTCT GGATAAAACG GAAGTATGGA TCTGCCCTTT GACCAATCCG    660
   GACGGAGCAT ACAGAGCCGG AAACCACACC GTACAAGGAG CTACTCGCTA CAATGCCAAC    720
   AATGTCGATT TGAACCGTAA CTTCAAGGAT GATGTAGCCG GTGATCACCC CGATGGAAAA    780
   CCTTGGCAGC CGGAGGCAAC TGCATTCATG GATTTGGAAG GAAACACCTC TTTCGTGCTC    840
   GGTGCCAATA TACATGGAGG AACAGAGGTG GTGAACTATC CATGGGATAA TAAAAAAGAA    900
   AGACATGCAG ACGATGAGTG GTACAAACTG ATCAGTCGCA ACTACGCAGC CGCTTGTCAG    960
   AGTATTTCCG CCAGCTACAT GACCTCCGAA ACCAATTCGG GAATCATCAA CGGTTCAGAC   1020
   TGGTATGTAA TTCGCGGAAG TCGTCAGGAC AATGCAAATT ATTTCCATCG TCTGCGAGAA   1080
   ATTACCCTTG AAATCAGCAA CACGAAGTTG GTGCCGGCCT CTCAACTTCC AAAGTATTGG   1140
   AATCTGAACA AAGAATCTCT GCTTGCTCTG ATCGAAGAAT CCTTATACGG CATCCATGGT   1200
   ACAGTGACTT CCGCTGCGAA CGGACAGCCT CTCAAATGCC AGATCTTGAT AGAAAACCAT   1260
   GACAAGCGCA ACTCCGATGT TTACTCCGAT GCTACCACAG GCTACTACGT ACGTCCTATC   1320
   AAAGCCGGCA CTTATACGGT GAAATACAAA GCCGAGGGTT ATCCTGAGGC AACTCGTACC   1380
   ATTACGATCA AGGACAAAGA AACCGTCATC ATGGACATTG CATTGGGCAA CTCGGTTCCT   1440
   CTGCCTGTAC CCGATTTCAC AGCTTCTCCT ATGACCATCT CAGTAGGCGA AAGCGTCCAA   1500
   TTCCAAGATC AAACGACAAA TAACCCCACG AATTGGGAGT GGACGTTCGA AGGCGGACAG   1560
   CCTGCCATGA GTACAGAGCA GAATCCGCTC GTATCCTATA GTCATCCCGG TCAGTACGAC   1620
   GTTACGCTCA AAGTGTGGAA TGCAAGTGGT TCCAACACGA TTACGAAAGA AAAATTCATC   1680
   ACTGTCAATG CCGTTATGCC TGTAGCTGAA TTCGTCGGTA CCCCGACGGA AATAGAAGAG   1740
   GGCCAGACGG TATCTTTCCA AAACCAATCC ACCAATGCCA CCAACTACGT ATGGATATTC   1800
   GATGGCGGCA CTCCCGCTAC CAGTGAAGAC GAAAACCCGA CTGTGCTTTA CAGCAAAGCC   1860
   GGCCAATACG ATGTCACGCT CAAGGCGATC AGTGCTTCCG GTGAAACGGT GAAGACGAAA   1920
   GAAAAATACA TCACTGTCAA GAAAGCTCCG GTCCCTGCTC CGGTAGCCGA CTTCGAAGGA   1980
   ACACCTCGAA AAGTAAAGAA AGGCGAGACA GTTACTTTCA AAGACTTGTC TACGAACAAT   2040
   CCGACTTCAT GGCTTTGGGT GTTCGAAGGC GGCTCTCCTG CCACCAGCAC GGAGCAAAAC   2100
   CCGGTGGTCA CCTACAATGA AACAGGCAAG TACGATGTCC AGCTGACTGC CACCAACGAG   2160
   GGCGGAAGCA ATGTGAAGAA AGCAGAAGAC TACATTGAGG TTATCCTCGA TGACAGTGTC   2220
   GAGGACATAG TGGCACAGAC GGGTATCGTC ATTCGTCCGC AAAACGGAAC GAAGCAGATC   2280
   CTCATAGAAG CCAACGCTGC TATCAAAGCG ATCGTTCTCT ATGACATCAA TGGACGGGTC   2340
   GTACTCAAAA CTACTCCGAA TCAGCTCCGC TCGACCGTAG ATCTTTCCAT CCTGCCCGAA   2400
   GGAATCTACA CCATCAATAT CAAAACGGAA AAATCCGCTC GCACGGAAAA GATCCATATC   2460
   GGG                                                                 2463
(2) INFORMATION FOR SEQ ID NO:170
   (i) SEQUENCE CHARACTERISTICS:
      (A) LENGTH: 669 base pairs (B)TYPE: nucleic acid (C) STRANDEDNESS: double (D) TOPOLOGY: circular
   (ii) MOLECULE TYPE: DNA (genomic)
   (iii) HYPOTHETICAL: NO (iv) ANTI-SENSE: NO
   (vi) ORIGINAL SOURCE:
      (A) ORGANISM: PORYPHYROMONAS GINGIVALIS
   (ix) FEATURE:
      (A) NAME/KEY: misc_feature
      (B) LOCATION 1...669
   (xi) SEQUENCE DESCRIPTION: SEQ ID NO:170
   ATGAAGAAAT CAAGTGTAGT AGCCTCAGTT TTGGCCGTGG CTCTCGTGTT CGCCGGTTGC    60
   GGACTGAACA ATATGGCAAA AGGCGGCCTT ATCGGCGCCG GAGTAGGAGG TGCCATTGGT   120
   GCCGGAGTAG GTAACGTAGC CGGAAATACG GCTGTCGGTG CCATCGTCGG TACTGCAGTC   180
   GGTGGAGCAG CCGGTGCTCT CATCGGAAAG AAGATGGACA AGCAGAAAAA AGAACTGGAG   240
   GCCGCAGTAC CCGATGCTAC GATTCAGACA GTAAATGACG GAGAGGCTAT TCTGGTTACT   300
   TTCGATAGCG GTATCCTCTT TGCGACGAAC TCCAGCACTC TGAGTCCCAA CTCACGCACT   360
   GCGCTGACGA AGTTTGCTGC AAACATGAAC AAAAACCCCG ACACGGATAT TCGTATCGTA   420
   GGCCATACGG ACAATACCGG CTCCGACAAG ATCAACGATC CTCTGTCTGA GAGACGTGCA   480
   GCCAGCGTAT ATTCTTTCCT GAATTCTCAG GGTGTGAGTA TGTCCGCCAT GGCAGCCGAA   540
   GGGCGTGGGA GCCATGAACC GGTTGCAGAC AATAGCACAG TTGCCGGACG TTCGGCCAAC   600
   CGCCGTGTGG AGGTTTATAT CTTGCCGAAT GCCAAGATGA TCGAACAAGC ACAGCAAGGT   660
   ACGCTGAAG                                                          669
(2) INFORMATION FOR SEQ ID NO:171
   (i) SEQUENCE CHARACTERISTICS:
      (A) LENGTH: 1011 base pairs
      (B) TYPE: nucleic acid
      (C) STRANDEDNESS: double
      (D) TOPOLOGY: circular (ii) MOLECULE TYPE: DNA (genomic) (iii) HYPOTHETICAL: NO (iv) ANTI-SENSE: NO
   (vi) ORIGINAL SOURCE:
      (A) ORGANISM: PORYPHYROMONAS GINGIVALIS
   (ix) FEATURE:
      (A) NAME/KEY: misc_feature
      (B) LOCATION 1...1011
   (::i) SEQUENCE DESCRIPTION: SEQ ID NO:171
(2) INFORMATION FOR SEQ ID NO:261
   (1) SEQUENCE CHARACTERISTICS:
      (A) LENGTH: 1668 base pairs (B)TYPE: nucleic acid (C) STRANDEDHESS: double (D) TOPOLOGY: circular (ii) MOLECULE TYPE: DNA (genomic) (iii) HYPOTHETICAL: NO (iv) ANTI-SENSE: NO
   (vi) ORIGINAL SOURCE:
      (A) ORGANISH: PORYPHYROHONAS GINGIVALIS
   (ix) FEATURE:
      (A) NAME/KEY: misc_feature
      (B) LOCATION 1...1668
   (xi) SEQUENCE DESCRIPTION: SEQ ID NO:261
   ATGAAAAAGC TTTTACAGGC TAAAGCCTTG ATTCTGGCAT TGGGACTCTT CCAACTGCCC     60
   GCAATCGCCC AAACGCAAAT GCAAGCAGAC CGAACAAACG GTCAATTTGC AACAGAAGAG    120
   ATGCAACGAG CATTCCAGGA AACGAATCCC CCTGCAGGTC CTGTGCGTGC TATCGCTGAG    180
   TACGAACGCT CTGCAGCCGT TTTGGTACGC TACCCGTTCG GTATCCCGAT GGAATTGATC    240
   AAAGAGCTGG CCAAGAACGA CAAGGTGATT ACCATTGTGG CGAGTGAAAG CCAAAAAAAC    300
   ACCGTTATAA CCCAGTACAC CCAAAGCGGT GTGAATCTCT CTAATTGCGA TTTCATCATT    360
   GCGAAAACTG ACTCTTACTG GACACGCGAC TATACCGGTT GGTTCGCAAT GTACGATACG    420
   AACAAAGTAG GTCTCGTGGA CTTTATTTAT AACCGCCCTC GTCCTAACGA TGATGAATTC    480
   CCCAAATACG AAGCACAATA TCTGGGCATC GAGATGTTCG GGATGAAGCT CAAGCAGACC    540
   GGTGGCAACT ACATGACGGA CGGATATGGA TCCGCTGTGC AGTCACATAT CGCATATACG    600
   GAGAACTCCT CTCTGTCTCA AGCTCAAGTA AATCAAAAGA TGAAAGACTA TCTCGGCATC    660
   ACACATCATG ATGTGGTACA AGATCCGAAC GGCGAATATA TCAACCATGT GGACTGTTGG    720
   GGCAAGTATT TGGCACCGAA CAAAATCCTC ATCAGGAAAG TGCCTGACAA TCACCCTCAG    780
   CACCAAGCCC TGGAAGATAT GGCAGCCTAC TTCGCAGCAC AGACCTGCGC ATGGGGAACG    840
   AAGTACGAGG TATATCGCGC TTTGGCCACC AATGAACAAC CGTACACGAA CTCTCTGATT    900
   CTGAACAACA GGGTATTTGT TCCTGTCAAT GGCCCCGCCT CCGTGGACAA CGATGCTCTG    960
   AACGTCTATA AGACGGCAAT GCCCGGTTAC GAAATTATAG GTGTCAAAGG GGCTTCAGGA   1020
   ACACCTTGGT TAGGAACAGA TGCCCTGCAT TGTCGTACTC ACGAGGTAGC GGATAAGGGC   1080
   TATCTCTATA TCAAGCACTA CCCGATACTG GGCGAACAGG CAGGCCCTGA TTATAAGATC   1140
   GAAGCAGATG TCGTCTCATG CGCCAATGCT ACTATCTCGC CGGTACAATG TTACTATCGT   1200
   ATCAATGGTT CCGGTAGCTT TAAGGCTGCT GATATGACGA TGGAATCAAC AGGTCACTAT   1260
   ACTTATAGCT TTACAGGTCT TAACAAGAAT GATAAGGTAG AATACTATAT CTCTGCCGCT   1320
   GACAATAGTG GTCGCAAAGA GACTTATCCC TTTATCGGCG AACCTGATCC TTTCAAGTTT   1380
   ACGTGTATGA ACGAAACCAA TACATGTACT GTGACCGGAG CTGCCAAAGC TCTTCGTGCA   1440
   TGGTTCAACG CCGGTCGTTC AGAACTGGCT GTTTCGGTAA GTTTGAATAT TGCCGGCACA   1500
   TATCGGATAA AGCTTTATAA CACCGCAGGA GAAGAAGTCG CTGCAATGAC CAAGGAATTA   1560
   GTAGCAGGGA CGAGTGTCTT CAGTATGGAT GTGTATTCTC AGGCTCCGGG CACATATGTT   1620
   CTGGTTGTTG AAGGAAATGG AATCCGTGAG ACAATGAAAA TTCTCAAA                1668
(7) INFORMATION FOR SEQ ID NO:262
   (i) SEQUENCE CHARACTERISTICS:
      (A) LENGTH: 1284 base pairs
      (B) TYPE: nucleic acid
      (C) STRANDEDNESS: double
      (D) TOPOLOGY: circular
   (ii) MOLECULE TYPE: DNA (genomic)
   (iii) HYPOTHETICAL: NO
   (iv) ANTl-SENSE: NO
   (vi) ORIGINAL SOURCE:
      (A) ORGANISM: PORYPHYROMONAS GINGIVALIS
   (ix) FEATURE:
      (A) NAME/KEY: misc_feature
      (B) LOCATION 1...1284
   (xi) SEQUENCE DESCRIPTION: SEQ ID NO:262
   ATGAAACTTT CATCTAAGAA AATCTTAGCA ATCATTGCAT TGCTGACGAT GGGACATGCT     60
   GTGCAGGCAC AGTTTGTTCC GGCTCCCACC ACAGGGATTC GCATGTCTGT CACTACAACC    120
   AAGGCCGTAG GCGAAAAAAT CGAATTGTTG GTTCATTCCA TAGAGAAGAA AGGCATCTGG    180
   ATCGATCTCA ATGGGGATGC CACTTACCAA CAAGGAGAGG AAATAACCGT ATTCGATGAG    240
   GCATACCACG AATACACGAT CGGGACGCAA ACCCTCACTA TCTATGGTAA TACGACCCGA    300
   TTGGGCTGTC GATCTACCGG TGCAACGGCT GTCGATGTAA CGAAAAACCC TAATCTGACC    360
   TATCTCGCAT GCCCGAAAAA TAATCTGAAA TCATTGGACT TGACGCAAAA CCCAAAGCTG    420
   CTGCGAGTTT GGTGCGACTC TAACGAAATA GAAAGTTTGG ACCTGAGTGG CAATCCGGCT    480
   TTGATCATCC TCGGCTGTGA CAGGAATAAG CTGACTGAGC TGAAGACCGA TAACAACCCC    540
   AAGTTGGCCT CTCTTTGGTG TTCTGATAAT AACCTGACGG AGTTGGAACT CAGTGCCAAT    600
   CCTCGTCTCA ATGATCTTTG GTGCTTCGGT AATCGGATCA CGAAACTCGA TCTGAGTGCC    660
   AATCCTCTAT TGGTAACACT TTGGTGCAGT GACAATGAGC TTTCGACCTT GGATCTTTCC    720
   AAGAATTCGG ACGTTGCTTA CCTTTGGTGT TCATCGAACA AACTTACATC CTTGAATCTG    780
   TCGGGGGTGA AGGGACTGAG TGTTTTGGTT TGTCATTCCA ATCAGATCGC AGGTGAAGAA    840
   ATGACGAAAG TGGTGAATGC TTTGCCCACA CTATCTCCCG GCGCAGGCGC TCAGAGCAAG    900
   TTCGTCGTTG TAGACCTCAA GGACACTGAT GAGAAGAATA TCTGTACCGT AAAGGATGTG    960
   GAAAAAGCTA AAAGTAAGAA CTGGCGAGTA TTTGACTTCA ACGGTGATTC TGACAATATG   1020
   CTTCCATACG AAGGAAGTCC GACATCGAAC TTGGCAGTAG ATGCTCCCAC TGTCAGGATA   1080
   TATCCCAATC CGGTAGGAAG ATATGCGCTC GTCGAGATCC CCGAGTCTCT TTTAGGGCAG   1140
   GAAGCTGCTT TATACGATAT GAATGGGGTA AAAGTCTATA GTTTCGCGGT AGAGTCTCTT   1200
   CGTCAGAACA TTGACCTGAC ACATCTTCCC GACGGCACTT ATTTCTTCCG TCTCGATAAC   1260
   TATACCACTA AGCTCATCAA ACAG                                          1284
(2) INFORMATION FOR SEQ ID NO: 265
   (i) SEQUENCE CHARACTERISTICS:
      (A) LENGTH: 454 amino acids
      (B) TYPE: amino acid
      (D) TOPOLOGY: linear
   (ii) MOLECULE TYPE: protein
   (iii) HYPOTHETICAL: YES
   (vi) ORIGINAL SOURCE:
      (A) ORGANISH: Porphyromonas gingivalis
   (ix) FEATURE:
      (A) NAME/KEY: misc_feature
      (B) LOCATION 1...454
   (xi) SEQUENCE DESCRIPTION: SEQ ID NO: 265
   Phe Cys Val Met Ala Lys Val Ile Lys Thr Lys Lys Gly Leu Ala Leu
   1               5                   10                  15
   Asn Leu Lys Gly Lys Pro Leu Pro Glu Met Leu Ala Glu Pro Ala Gln
               20                  25                  30
   Ser Pro Thr Tyr Ala Val Val Pro Asp Asp Phe Glu Gly Val Ile Pro
           35                  40                  45
   Lys Val Thr Ala Arg Pro Gly Asp Lys Val Arg Ala Gly Ser Ala Leu
       50                  55                  60
   Met His His Lys Ala Tyr Pro Glu Met Lys Phe Thr Ser Pro Val Ser
   65                  70                  75                  80
   Gly Glu Val Ile Ala Val Asn Arg Gly Ala Lys Arg Lys Val Leu Ser
                   85                  90                  95
   Ile Glu Val Lys Pro Asp Gly Leu Asn Glu Tyr Glu Ser Phe Pro Val
               100                 105                 110
   Gly Asp Pro Ser Ala Leu Ser Ala Glu Gln Ile Lys Glu Leu Leu Leu
           115                 120                 125
   Ser Ser Gly Met Trp Gly Phe Ile Lys Gln Arg Pro Tyr Asp Ile Val
       130                 135                 140
   Ala Thr Pro Asp Ile Ala Pro Arg Asp Ile Tyr Ile Thr Ala Asn Phe
   145                 150                 155                 160
   Thr Ala Pro Leu Ala Pro Asp Phe Asp Phe Ile Val Arg Gly Glu Glu
                   165                 170                 175
   Arg Ala Leu Gln Thr Ala Ile Asp Ala Leu Ala Lys Leu Thr Thr Gly
               180                 185                 190
   Lys Val Tyr Val Gly Leu Lys Pro Gly Ser Ser Leu Gly Leu His Asn
           195                 200                 205
   Ala Glu Ile Val Glu Val His Gly Pro His Pro Ala Gly Asn Val Gly
       210                 215                 220
   Val Leu Ile Asn His Thr Lys Pro Ile Asn Arg Gly Glu Thr Val Trp
   225                 230                 235                 240
   Thr Leu Lys Ala Thr Asp Leu Ile Val Ile Gly Arg Phe Leu Leu Thr
                   245                 250                 255
   Gly Lys Ala Asp Phe Thr Arg Met Ile Ala Met Thr Gly Ser Asp Ala
               260                 265                 270
   Ala Ala His Gly Tyr Val Arg Ile Met Pro Gly Cys Asn Val Phe Ala
           275                 280                 285
   Ser Phe Pro Gly Arg Leu Thr Ile Lys Glu Ser His Glu Arg Val Ile
       290                 295                 300
   Asp Gly Asn Val Leu Thr Gly Lys Lys Leu Cys Glu Lys Glu Pro Phe
   305                 310                 315                 320
   Leu Ser Ala Arg Cys Asp Gln Ile Thr Val Ile Pro Glu Gly Asp Asp
                   325                 330                 335
   Val Asp Glu Leu Phe Gly Trp Ala Ala Pro Arg Leu Asp Gln Tyr Ser
               340                 345                 350
   Met Ser Arg Ala Tyr Phe Ser Trp Leu Gln Gly Lys Asn Lys Glu Tyr
           355                 360                 365
   Val Leu Asp Ala Arg Ile Lys Gly Gly Glu Arg Ala Met Ile Met Ser
       370                 375                 380
   Asn Glu Tyr Asp Arg Val Phe Pro Met Asp Ile Tyr Pro Glu Tyr Leu
   385                 390                 395                 400
   Leu Lys Ala Ile Ile Ala Phe Asp Ile Asp Lys Met Glu Asp Leu Gly
                   405                 410                 415
   Ile Tyr Glu Val Ala Pro Glu Asp Phe Ala Thr Cys Glu Phe Val Asp
               420                 425                 430
   Thr Ser Lys Ile Glu Leu Gln Arg Ile Val Arg Glu Gly Leu Asp Met
           435                 440                 445
   Leu Tyr Lys Glu Met Asn
       450
(2) INFORMATION FOR SEQ ID NO: 301
   (i) SEQUENCE CHARACTERISTICS:
      (A) LENGTH: 869 amino acids
      (B) TYPE: amino acid
      (D) TOPOLOGY: linear (ii) MOLECULE TYPE: protein (iii) HYPOTHETICAL: YES
   (vi) ORIGINAL SOURCE:
      (A) ORGANISM: Porphyromonas gingivalis
   (ix) FEATURE:
      (A) NAME/KEY: misc_feature
      (B) LOCATION 1...869
   (xi) SEQUENCE DESCRIPTION: SEQ ID NO: 301
   Trp His Arg Asn Ile Phe Ile Phe Ala Ser Thr Phe Ser Pro Lys Asn
   1               5                   10                  15
   Met Leu Pro Leu Pro Tyr Arg Tyr Ala Lys Thr Glu His Leu Phe Leu
               20                  25                  30
   Ala Lys Gly Tyr Cys Lys Asn Pro Ile Thr Asn Ile Ile Ile Leu Phe
           35                  40                  45
   Met Lys Lys Lys Asn Phe Leu Leu Leu Gly Ile Phe Val Ala Leu Leu
       50                  55                  60
   Thr Phe Ile Gly Ser Met Gln Ala Gln Gln Ala Lys Asp Tyr Phe Asn
   65                  70                  75                  80
   Phe Asp Glu Arg Gly Glu Ala Tyr Phe Ser Phe Lys Val Pro Asp Arg
                   85                  90                  95
   Ala Val Leu Gln Glu Leu Ala Leu Ile Met Ser Ile Asp Glu Phe Asp
               100                 105                 110
   Pro Val Thr Asn Glu Ala Ile Ala Tyr Ala Ser Glu Glu Glu Phe Glu
           115                 120                 125
   Ala Phe Leu Arg Tyr Gly Leu Lys Pro Thr Phe Leu Thr Pro Pro Ser
       130                 135                 140
   Met Gln Arg Ala Val Glu Met Phe Asp Tyr Arg Ser Gly Glu Lys Tyr
   145                 150                 155                 160
   Glu Trp Asn Ala Tyr Pro Thr Tyr Glu Ala Tyr Ile Ser Met Met Glu
                   165                 170                 175
   Glu Phe Gln Thr Lys Tyr Pro Ser Leu Cys Thr Thr Ser Val Ile Gly
               180                 185                 190
   Lys Ser Val Lys Asp Arg Lys Leu Met Ile Cys Lys Leu Thr Ser Ser
           195                 200                 205
   Ala Asn Thr Gly Lys Lys Pro Arg Val Leu Tyr Thr Ser Thr Met His
       210                 215                 220
   Gly Asp Glu Thr Thr Gly Tyr Val Val Leu Leu Arg Leu Ile Asp His
   225                 230                 235                 240
   Leu Leu Ser Asn Tyr Glu Ser Asp Pro Arg Ile Lys Asn Ile Leu Asp
                   245                 250                 255
   Lys Thr Glu Val Trp Ile Cys Pro Leu Thr Asn Pro Asp Gly Ala Tyr
   260 265 270
   Arg Ala Gly Asn His Thr Val Gln Gly Ala Thr Arg Tyr Asn Ala Asn
   275 280 285
   Asn Val Asp Leu Asn Arg Asn Phe Lys Asp Asp Val Ala Gly Asp His
   290 295 300
   Pro Asp Gly Lys Pro Trp Gln Pro Glu Ala Thr Ala Phe Met Asp Leu
   305 310 315 320
   Glu Gly Asn Thr Ser Phe Val Leu Gly Ala Asn Ile His Gly Gly Thr
   325 330 335
   Glu Val Val Asn Tyr Pro Trp Asp Asn Lys Lys Glu Arg His Ala Asp
   340 345 350
   Asp Glu Trp Tyr Lys Leu Ile Ser Arg Asn Tyr Ala Ala Ala Cys Gln
   355 360 365
   Ser Ile Ser Ala Ser Tyr Met Thr Ser Glu Thr Asn Ser Gly Ile Ile
   370 375 380
   Asn Gly Ser Asp Trp Tyr Val Ile Arg Gly Ser Arg Gln Asp Asn Ala
   385 390 395 400
   Asn Tyr Phe His Arg Leu Arg Glu Ile Thr Leu Glu Ile Ser Asn Thr
   405 410 415
   Lys Leu Val Pro Ala Ser Gln Leu Pro Lys Tyr Trp Asn Leu Asn Lys
   420 425 430
   Glu Ser Leu Leu Ala Leu Ile Glu Glu Ser Leu Tyr Gly Ile His Gly
   435 440 445
   Thr Val Thr Ser Ala Ala Asn Gly Gln Pro Leu Lys Cys Gln Ile Leu
   450 455 460
   Ile Glu Asn His Asp Lys Arg Asn Ser Asp Val Tyr Ser Asp Ala Thr
   465 470 475 480
   Thr Gly Tyr Tyr Val Arg Pro Ile Lys Ala Gly Thr Tyr Thr Val Lys
   485 490 495
   Tyr Lys Ala Glu Gly Tyr Pro Glu Ala Thr Arg Thr Ile Thr Ile Lys
   500 505 510
   Asp Lys Glu Thr Val Ile Met Asp Ile Ala Leu Gly Asn Ser Val Pro
   515 520 525
   Leu Pro Val Pro Asp Phe Thr Ala Ser Pro Met Thr Ile Ser Val Gly
   530 535 540
   Glu Ser Val Gln Phe Gln Asp Gln Thr Thr Asn Asn Pro Thr Asn Trp
   545 550 555 560
   Glu Trp Thr Phe Glu Gly Gly Gln Pro Ala Met Ser Thr Glu Gln Asn
   565 570 575
   Pro Leu Val Ser Tyr Ser His Pro Gly Gln Tyr Asp Val Thr Leu Lys
   580 585 590
   Val Trp Asn Ala Ser Gly Ser Asn Thr Ile Thr Lys Glu Lys Phe Ile
   595 600 605
   Thr Val Asn Ala Val Met Pro Val Ala Glu Phe Val Gly Thr Pro Thr
   610 615 620
   Glu Ile Glu Glu Gly Gln Thr Val Ser Phe Gln Asn Gln Ser Thr Asn
   625 630 635 640
   Ala Thr Asn Tyr Val Trp Ile Phe Asp Gly Gly Thr Pro Ala Thr Ser
   645 650 655
   Glu Asp Glu Asn Pro Thr Val Leu Tyr Ser Lys Ala Gly Gln Tyr Asp
   660 665 670
   Val Thr Leu Lys Ala Ile Ser Ala Ser Gly Glu Thr Val Lys Thr Lys
   675 680 685
   Glu Lys Tyr Ile Thr Val Lys Lys Ala Pro Val Pro Ala Pro Val Ala
   690 695 700
   Asp Phe Glu Gly Thr Pro Arg Lys Val Lys Lys Gly Glu Thr Val Thr
   705 710 715 720
   Phe Lys Asp Leu Ser Thr Asn Asn Pro Thr Ser Trp Leu Trp Val Phe
   725 730 735
   Glu Gly Gly Ser Pro Ala Thr Ser Thr Glu Gln Asn Pro Val Val Thr
   740 745 750
   Tyr Asn Glu Thr Gly Lys Tyr Asp Val Gln Leu Thr Ala Thr Asn Glu
   755 760 765
   Gly Gly Ser Asn Val Lys Lys Ala Glu Asp Tyr Ile Glu Val Ile Leu
   770 775 780
   Asp Asp Ser Val Glu Asp Ile Val Ala Gln Thr Gly Ile Val Ile Arg
   785 790 795 800
   Pro Gln Asn Gly Thr Lys Gln Ile Leu Ile Glu Ala Asn Ala Ala Ile
   805 810 815
   Lys Ala Ile Val Leu Tyr Asp Ile Asn Gly Arg Val Val Leu Lys Thr
   820 825 830
   Thr Pro Asn Gln Leu Arg Ser Thr Val Asp Leu Ser Ile Leu Pro Glu
   835 840 845
   Gly Ile Tyr Thr Ile Asn Ile Lys Thr Glu Lys Ser Ala Arg Thr Glu
           850                    855                     860
   Lys Ile His Ile Gly
   865
(2) INFORMATION FOR SEQ ID NO: 309
   (i) SEQUENCE CHARACTERISTICS:
      (A) LENGTH: 230 amino acids
      (B) TYPE: amino acid
      (D) TOPOLOGY: linear (ii) MOLECULE TYPE: protein (iii) HYPOTHETICAL: YES
   (vi) ORIGINAL SOURCE:
      (A) ORGANISM: Porphyromonas gingivalis
   (ix) FEATURE:
      (A) NAME/KEY: misc_feature
      (B) LOCATION 1...230
   (xi) SEQUENCE DESCRIPTION: SEQ ID NO: 309
   Thr Asn Lys Lys Glu Glu Thr Met Lys Lys Ser Ser Val Val Ala Ser
   1               5                   10                  15
   Val Leu Ala Val Ala Leu Val Phe Ala Gly Cys Gly Leu Asn Asn Met
               20                  25                  30
   Ala Lys Gly Gly Leu Ile Gly Ala Gly Val Gly Gly Ala Ile Gly Ala
           35                  40                  45
   Gly Val Gly Asn Val Ala Gly Asn Thr Ala Val Gly Ala Ile Val Gly
       50                  55                  60
   Thr Ala Val Gly Gly Ala Ala Gly Ala Leu Ile Gly Lys Lys Met Asp
   65                  70                  75                  80
   Lys Gln Lys Lys Glu Leu Glu Ala Ala Val Pro Asp Ala Thr Ile Gln
                   85                  90                  95
   Thr Val Asn Asp Gly Glu Ala Ile Leu Val Thr Phe Asp Ser Gly Ile
               100                 105                 110
   Leu Phe Ala Thr Asn Ser Ser Thr Leu Ser Pro Asn Ser Arg Thr Ala
           115                 120                 125
   Leu Thr Lys Phe Ala Ala Asn Met Asn Lys Asn Pro Asp Thr Asp Ile
       130                 135                 140
   Arg Ile Val Gly His Thr Asp Asn Thr Gly Ser Asp Lys Ile Asn Asp
   145                 150                 155                 160
   Pro Leu Ser Glu Arg Arg Ala Ala Ser Val Tyr Ser Phe Leu Asn Ser
                   165                 170                 175
   Gln Gly Val Ser Met Ser Arg Met Ala Ala Glu Gly Arg Gly Ser His
               180                 185                 190
   Glu Pro Val Ala Asp Asn Ser Thr Val Ala Gly Arg Ser Ala Asn Arg
           195                 200                 205
   Arg Val Glu Val Tyr Ile Leu Pro Asn Ala Lys Met Ile Glu Gln Ala
       210                 215                 220
   Gln Gln Gly Thr Leu Lys
   225                 230
(2) INFORMATION FOR SEQ ID NO: 310
   (i) SEQUENCE CHARACTERISTICS:
      (A) LENGTH: 342 amino acids
      (B) TYPE: amino acid
      (D) TOPOLOGY: linear
   (ii) MOLECULE TYPE: protein
   (iii) HYPOTHETICAL: YES
   (vi) ORIGINAL SOURCE:
      (A) ORGANISM: Porphyromonas gingivalis
   (ix) FEATURE:
      (A) NAME/KEY: misc_feature
      (B) LOCATION 1...342
   (xi) SEQUENCE DESCRIPTION: SEQ ID NO: 310
   Asn Arg Asn Arg Asn Met Ser Lys Lys Ser Ile Leu Leu Leu Cys Cys
   1               5                   10                  15
   Ser Leu Cys Phe Ile Ser Ala Thr Lys Ala Val Thr Pro Val Arg Asn
               20                  25                  30
   Val Arg Asn Ser Gln Val Asn Ser Lys Ala Lys Thr Glu Arg Thr Lys
           35                  40                  45
   Pro Ser Asp Ser Val Arg Tyr Ile Ser Asn Met Ile Ala Asp Arg Leu
       50                  55                  60
   Glu Phe Arg Asn Lys Ile Ser Ser Glu Lys Glu Val Arg Lys Ala Glu
   65                  70                  75                  80
   Tyr Glu Asn Arg Leu Ala Met Glu Ala Leu Asn Tyr Pro Ala Ile Asp
                   85                  90                  95
   Leu Tyr Gly Glu Asp Ser Trp Ser Glu Tyr Val Asn Pro Phe Val Gly
               100                 105                 110
   Ala Gly Thr Asp Val Glu Ile Pro Asn Ser Tyr Asp Ile Asp Cys Ser
           115                 120                 125
   Ser Phe Val Met Pro Val Glu Asp Lys Gln Val Thr Ser Gln Phe Gly
       130                 135                 140
   Tyr Arg Arg Arg Phe Gly Arg Met His Tyr Gly Ile Asp Leu Ser Val
   145                 150                 155                 160
   Asn Arg Gly Asp Thr Ile Arg Ala Ala Phe Asp Gly Lys Val Arg Val
                   165                 170                 175
   Arg Ser Tyr Glu Ala Arg Gly Tyr Gly Tyr Tyr Ile Val Leu Arg His
               180                 185                 190
   Pro Asn Gly Leu Glu Thr Val Tyr Gly His Met Ser Arg Gln Leu Val
           195                 200                 205
   Asp Glu Asn Gln Ile Val Arg Ala Gly Gln Pro Ile Gly Leu Gly Gly
       210                 215                 220
   Ser Thr Gly Arg Ser Thr Gly Pro His Leu His Phe Glu Thr Arg Phe
   225                 230                 235                 240
   Met Gly Ile Pro Ile Asn Pro Ser Thr Ile Ile Asp Phe Asp Asn Gly
                   245                 250                 255
   Val Pro Leu Arg Asp Ile Tyr Thr Phe Lys Arg Gly Ser Asn Ser Arg
               260                 265                 270
   Tyr Ala Lys Ala Ser Lys Thr Ser Ser Arg Tyr Ala Lys Lys Gly Lys
           275                 280                 285
   Lys Gly Arg Gln Ala Ser Ser Pro Met Thr Tyr Arg Ile Lys Lys Gly
       290                 295                 300
   Asp Thr Leu Glu Thr Ile Ala Lys Arg His Gly Thr Ser Val Gln Lys
   305                 310                 315                 320
   Leu Cys Ala Thr Asn Gly Ile Gly Lys Ser Lys Ile Leu Thr Pro Gly
                   325                 330                 335
   Lys Ala Leu Arg Ile Lys
               340
(2) INFORMATION FOR SEQ ID NO: 382
   (i) SEQUENCE CHARACTERISTICS:
      (A) LENGTH: 563 amino acids
      (B) TYPE: aminoacid
      (D) TOPOLOGY: linear (ii) MOLECULE TYPE: protein (iii) HYPOTHETICAL: YES
   (vi) ORIGINAL SOURCE:
      (A) ORGANISM: Porphyromonas gingivalis
   (ix) FEATURE:
      (A) NAME/KEY: misc_feature
      (B) LOCATION 1...563
   (xi) SEQUENCE DESCRIPTION: SEQ ID NO: 382
   His His Lys Thr Tyr Gln Thr Met Lys Lys Leu Leu Gln Ala Lys Ala
   1               5                   10                  15
   Leu Ile Leu Ala Leu Gly Leu Phe Gln Leu Pro Ala Ile Ala Gln Thr
               20                  25                  30
   Gln Met Gln Ala Asp Arg Thr Asn Gly Gln Phe Ala Thr Glu Glu Met
           35                  40                  45
   Gln Arg Ala Phe Gln Glu Thr Asn Pro Pro Ala Gly Pro Val Arg Ala
       50                  55                  60
   Ile Ala Glu Tyr Glu Arg Ser Ala Ala Val Leu Val Arg Tyr Pro Phe
   65                  70                  75                  80
   Gly Ile Pro Met Glu Leu Ile Lys Glu Leu Ala Lys Asn Asp Lys Val
                   85                  90                  95
   Ile Thr Ile Val Ala Ser Glu Ser Gln Lys Asn Thr Val Ile Thr Gln
               100                 105                 110
   Tyr Thr Gln Ser Gly Val Asn Leu Ser Asn Cys Asp Phe Ile Ile Ala
           115                 120                 125
   Lys Thr Asp Ser Tyr Trp Thr Arg Asp Tyr Thr Gly Trp Phe Ala Met
       130                 135                 140
   Tyr Asp Thr Asn Lys Val Gly Leu Val Asp Phe Ile Tyr Asn Arg Pro
   145                 150                 155                 160
   Arg Pro Asn Asp Asp Glu Phe Pro Lys Tyr Glu Ala Gln Tyr Leu Gly
                   165                 170                 175
   Ile Glu Met Phe Gly Met Lys Leu Lys Gln Thr Gly Gly Asn Tyr Met
               180                 185                 190
   Thr Asp Gly Tyr Gly Ser Ala Val Gln Ser His Ile Ala Tyr Thr Glu
           195                 200                 205
   Asn Ser Ser Leu Ser Gln Ala Gln Val Asn Gln Lys Met Lys Asp Tyr
       210                 215                 220
   Leu Gly Ile Thr His His Asp Val Val Gln Asp Pro Asn Gly Glu Tyr
   225                 230                 235                 240
   Ile Asn His Val Asp Cys Trp Gly Lys Tyr Leu Ala Pro Asn Lys Ile
                   245                 250                 255
   Leu Ile Arg Lys Val Pro Asp Asn His Pro Gln His Gln Ala Leu Glu
               260                 265                 270
   Asp Met Ala Ala Tyr Phe Ala Ala Gln Thr Cys Ala Trp Gly Thr Lys
           275                 280                 285
   Tyr Glu Val Tyr Arg Ala Leu Ala Thr Asn Glu Gln Pro Tyr Thr Asn
       290                 295                 300
   Ser Leu Ile Leu Asn Asn Arg Val Phe Val Pro Val Asn Gly Pro Ala
   305                 310                 315                 320
   Ser Val Asp Asn Asp Ala Leu Asn Val Tyr Lys Thr Ala Met Pro Gly
                   325                 330                 335
   Tyr Glu Ile Ile Gly Val Lys Gly Ala Ser Gly Thr Pro Trp Leu Gly
               340                 345                 350
   Thr Asp Ala Leu His Cys Arg Thr His Glu Val Ala Asp Lys Gly Tyr
           355                 360                 365
   Leu Tyr Ile Lys His Tyr Pro Ile Leu Gly Glu Gln Ala Gly Pro Asp
       370                 375                 380
   Tyr Lys Ile Glu Ala Asp Val Val Ser Cys Ala Asn Ala Thr Ile Ser
   385                 390                 395                 400
   Pro Val Gln Cys Tyr Tyr Arg Ile Asn Gly Ser Gly Ser Phe Lys Ala
                   405                 410                 415
   Ala Asp Met Thr Met Glu Ser Thr Gly His Tyr Thr Tyr Ser Phe Thr
               420                 425                 430
   Gly Leu Asn Lys Asn Asp Lys Val Glu Tyr Tyr Ile Ser Ala Ala Asp
           435                 440                 445
   Asn Ser Gly Arg Lys Glu Thr Tyr Pro Phe Ile Gly Glu Pro Asp Pro
       450                 455                 460
   Phe Lys Phe Thr Cys Met Asn Glu Thr Asn Thr Cys Thr Val Thr Gly
   465                 470                 475                 480
   Ala Ala Lys Ala Leu Arg Ala Trp Phe Asn Ala Gly Arg Ser Glu Leu
                   485                 490                 495
   Ala Val Ser Val Ser Leu Asn Ile Ala Gly Thr Tyr Arg Ile Lys Leu
               500                 505                 510
   Tyr Asn Thr Ala Gly Glu Glu Val Ala Ala Met Thr Lys Glu Leu Val
           515                 520                 525
   Ala Gly Thr Ser Val Phe Ser Met Asp Val Tyr Ser Gln Ala Pro Gly
       530                 535                 540
   Thr Tyr Val Leu Val Val Glu Gly Asn Gly Ile Arg Glu Thr Met Lys
   545                 550                 555                 560
   Ile Leu Lys
(2) INFORMATION FOR SEQ ID NO: 383
   (i) SEQUENCE CHARACTERISTICS:
      (A) LENGTH: 437 amino acids
      (B) TYPE: amino acid
      (D) TOPOLOGY: linear (ii) MOLECULE TYPE: protein (iii) HYPOTHETICAL: YES
   (vi) ORIGINAL SOURCE:
      (A) ORGANISM: Porphyromonas gingivalis
   (ix) FEATURE:
      (A) NAME/KEY: misc_feature
      (B) LOCATION 1...437
   (xi) SEQUENCE DESCRIPTION: SEQ ID NO: 383
   Thr Thr Asn Arg Lys Pro Asn Thr Asn Met Lys Leu Ser Ser Lys Lys
   1               5                   10                  15
   Ile Leu Ala Ile Ile Ala Leu Leu Thr Met Gly His Ala Val Gln Ala
               20                  25                  30
   Gln Phe Val Pro Ala Pro Thr Thr Gly Ile Arg Met Ser Val Thr Thr
           35                  40                  45
   Thr Lys Ala Val Gly Glu Lys Ile Glu Leu Leu Val His Ser Ile Glu
       50                  55                  60
   Lys Lys Gly Ile Trp Ile Asp Leu Asn Gly Asp Ala Thr Tyr Gln Gln
   65                  70                  75                  80
   Gly Glu Glu Ile Thr Val Phe Asp Glu Ala Tyr His Glu Tyr Thr Ile
                   85                  90                  95
   Gly Thr Gln Thr Leu Thr Ile Tyr Gly Asn Thr Thr Arg Leu Gly Cys
               100                 105                 110
   Arg Ser Thr Gly Ala Thr Ala Val Asp Val Thr Lys Asn Pro Asn Leu
           115                 120                 125
   Thr Tyr Leu Ala Cys Pro Lys Asn Asn Leu Lys Ser Leu Asp Leu Thr
       130                 135                 140
   Gln Asn Pro Lys Leu Leu Arg Val Trp Cys Asp Ser Asn Glu Ile Glu
   145                 150                 155                 160
   Ser Leu Asp Leu Ser Gly Asn Pro Ala Leu Ile Ile Leu Gly Cys Asp
                   165                 170                 175
   Arg Asn Lys Leu Thr Glu Leu Lys Thr Asp Asn Asn Pro Lys Leu Ala
               180                 185                 190
   Ser Leu Trp Cys Ser Asp Asn Asn Leu Thr Glu Leu Glu Leu Ser Ala
           195                 200                 205
   Asn Pro Arg Leu Asn Asp Leu Trp Cys Phe Gly Asn Arg Ile Thr Lys
       210                 215                 220
   Leu Asp Leu Ser Ala Asn Pro Leu Leu Val Thr Leu Trp Cys Ser Asp
   225                 230                 235                 240
   Asn Glu Leu Ser Thr Leu Asp Leu Ser Lys Asn Ser Asp Val Ala Tyr
                   245                 250                 255
   Leu Trp Cys Ser Ser Asn Lys Leu Thr Ser Leu Asn Leu Ser Gly Val
               260                 265                 270
   Lys Gly Leu Ser Val Leu Val Cys His Ser Asn Gln Ile Ala Gly Glu
           275                 280                 285
   Glu Met Thr Lys Val Val Asn Ala Leu Pro Thr Leu Ser Pro Gly Ala
       290                 295                 300
   Gly Ala Gln Ser Lys Phe Val Val Val Asp Leu Lys Asp Thr Asp Glu
   305                 310                 315                 320
   Lys Asn Ile Cys Thr Val Lys Asp Val Glu Lys Ala Lys Ser Lys Asn
                   325                 330                 335
   Trp Arg Val Phe Asp Phe Asn Gly Asp Ser Asp Asn Met Leu Pro Tyr
               340                 345                 350
   Glu Gly Ser Pro Thr Ser Asn Leu Ala Val Asp Ala Pro Thr Val Arg
           355                 360                 365
   Ile Tyr Pro Asn Pro Val Gly Arg Tyr Ala Leu Val Glu Ile Pro Glu
       370                 375                 380
   Ser Leu Leu Gly Gln Glu Ala Ala Leu Tyr Asp Met Asn Gly Val Lys
   385                 390                 395                 400
   Val Tyr Ser Phe Ala Val Glu Ser Leu Arg Gln Asn Ile Asp Leu Thr
                   405                 410                 415
   His Leu Pro Asp Gly Thr Tyr Phe Phe Arg Leu Asp Asn Tyr Thr Thr
               420                 425                 430
   Lys Leu Ile Lys Gln
           435
(2) INFORMATION FOR SEQ ID NO: 386
   (i) SEQUENCE CHARACTERISTICS:
      (A) LENGTH: 451 amino acids
      (B) TYPE: amino acid
      (D) TOPOLOGY: linear (ii) MOLECULE TYPE: protein (iii) HYPOTHETICAL: YES
   (vi) ORIGINAL SOURCE:
      (A) ORGANISM: Porphyromonas gingivalis
   (ix) FEATURE:
      (A) NAME/KEY: misc_feature
      (B) LOCATION 1... 451
   (xi) SEQUENCE DESCRIPTION: SEQ ID NO: 386
   Met Ala Lys Val Ile Lys Thr Lys Lys Gly Leu Ala Leu Asn Leu Lys
   1               5                   10                  15
   Gly Lys Pro Leu Pro Glu Met Leu Ala Glu Pro Ala Gln Ser Pro Thr
               20                  25                  30
   Tyr Ala Val Val Pro Asp Asp Phe Glu Gly Val Ile Pro Lys Val Thr
           35                  40                  45
   Ala Arg Pro Gly Asp Lys Val Arg Ala Gly Ser Ala Leu Met His His
       50                  55                  60
   Lys Ala Tyr Pro Glu Met Lys Phe Thr Ser Pro Val Ser Gly Glu Val
   65                  70                  75                  80
   Ile Ala Val Asn Arg Gly Ala Lys Arg Lys Val Leu Ser Ile Glu Val
                   85                  90                  95
   Lys Pro Asp Gly Leu Asn Glu Tyr Glu Ser Phe Pro Val Gly Asp Pro
               100                 105                 110
   Ser Ala Leu Ser Ala Glu Gln Ile Lys Glu Leu Leu Leu Ser Ser Gly
           115                 120                 125
   Met Trp Gly Phe Ile Lys Gln Arg Pro Tyr Asp Ile Val Ala Thr Pro
       130                 135                 140
   Asp Ile Ala Pro Arg Asp Ile Tyr Ile Thr Ala Asn Phe Thr Ala Pro
   145                 150                 155                 160
   Leu Ala Pro Asp Phe Asp Phe Ile Val Arg Gly Glu Glu Arg Ala Leu
                   165                 170                 175
   Gln Thr Ala Ile Asp Ala Leu Ala Lys Leu Thr Thr Gly Lys Val Tyr
               180                 185                 190
   Val Gly Leu Lys Pro Gly Ser Ser Leu Gly Leu His Asn Ala Glu Ile
           195                 200                 205
   Val Glu Val His Gly Pro His Pro Ala Gly Asn Val Gly Val Leu Ile
       210                 215                 220
   Asn His Thr Lys Pro Ile Asn Arg Gly Glu Thr Val Trp Thr Leu Lys
   225                 230                 235                 240
   Ala Thr Asp Leu Ile Val Ile Gly Arg Phe Leu Leu Thr Gly Lys Ala
                   245                 250                 255
   Asp Phe Thr Arg Met Ile Ala Met Thr Gly Ser Asp Ala Ala Ala His
               260                 265                 270
   Gly Tyr Val Arg Ile Met Pro Gly Cys Asn Val Phe Ala Ser Phe Pro
           275                 280                 285
   Gly Arg Leu Thr Ile Lys Glu Ser His Glu Arg Val Ile Asp Gly Asn
       290                 295                 300
   Val Leu Thr Gly Lys Lys Leu Cys Glu Lys Glu Pro Phe Leu Ser Ala
   305                 310                 315                 320
   Arg Cys Asp Gln Ile Thr Val Ile Pro Glu Gly Asp Asp Val Asp Glu
                   325                 330                 335
   Leu Phe Gly Trp Ala Ala Pro Arg Leu Asp Gln Tyr Ser Met Ser Arg
               340                 345                 350
   Ala Tyr Phe Ser Trp Leu Gln Gly Lys Asn Lys Glu Tyr Val Leu Asp
           355                 360                 365
   Ala Arg Ile Lys Gly Gly Glu Arg Ala Met Ile Met Ser Asn Glu Tyr
       370                 375                 380
   Asp Arg Val Phe Pro Met Asp Ile Tyr Pro Glu Tyr Leu Leu Lys Ala
   385                 390                 395                 400
   Ile Ile Ala Phe Asp Ile Asp Lys Met Glu Asp Leu Gly Ile Tyr Glu
                   405                 410                 415
   Val Ala Pro Glu Asp Phe Ala Thr Cys Glu Phe Val Asp Thr Ser Lys
               420                 425                 430
   Ile Glu Leu Gln Arg Ile Val Arg Glu Gly Leu Asp Met Leu Tyr Lys
           435                 440                 445
   Glu Met Asn
       450
(2) INFORMATION FOR SEQ ID NO: 426
   (j) SEQUENCE CHARACTERISTICS:
      (A) LENGTH: 821 amino acids
      (B) TYPE: amino acid
      (D) TOPOLOGY: linear (ii) MOLECULE TYPE: protein (iii) HYPOTHETICAL: YES
   (vi) ORIGINAL SOURCE:
      (A) ORGANISM: Porphyromonas gingivalis
   (ix) FEATURE:
      (A) NAME/KEY: misc_feature
      (B) LOCATION 1...821
   (xi) SEQUENCE DESCRIPTION: SEQ ID NO: 426
   Met Lys Lys Lys Asn Phe Leu Leu Leu Gly Ile Phe Val Ala Leu Leu
   1               5                   10                  15
   Thr Phe Ile Gly Ser Met Gln Ala Gln Gln Ala Lys Asp Tyr Phe Asn
               20                  25                  30
   Phe Asp Glu Arg Gly Glu Ala Tyr Phe Ser Phe Lys Val Pro Asp Arg
           35                  40                  45
   Ala Val Leu Gln Glu Leu Ala Leu Ile Met Ser Ile Asp Glu Phe Asp
       50                  55                  60
   Pro Val Thr Asn Glu Ala Ile Ala Tyr Ala Ser Glu Glu Glu Phe Glu
   65                  70                  75                  80
   Ala Phe Leu Arg Tyr Gly Leu Lys Pro Thr Phe Leu Thr Pro Pro Ser
                   85                  90                  95
   Met Gln Arg Ala Val Glu Met Phe Asp Tyr Arg Ser Gly Glu Lys Tyr
               100                 105                 110
   Glu Trp Asn Ala Tyr Pro Thr Tyr Glu Ala Tyr Ile Ser Met Met Glu
           115                 120                 125
   Glu Phe Gln Thr Lys Tyr Pro Ser Leu Cys Thr Thr Ser Val Ile Gly
       130                 135                 140
   Lys Ser Val Lys Asp Arg Lys Leu Met Ile Cys Lys Leu Thr Ser Ser
   145                 150                 155                 160
   Ala Asn Thr Gly Lys Lys Pro Arg Val Leu Tyr Thr Ser Thr Met His
                   165                 170                 175
   Gly Asp Glu Thr Thr Gly Tyr Val Val Leu Leu Arg Leu Ile Asp His
               180                 185                 190
   Leu Leu Ser Asn Tyr Glu Ser Asp Pro Arg Ile Lys Asn Ile Leu Asp
           195                 200                 205
   Lys Thr Glu Val Trp Ile Cys Pro Leu Thr Asn Pro Asp Gly Ala Tyr
       210                 215                 220
   Arg Ala Gly Asn His Thr Val Gln Gly Ala Thr Arg Tyr Asn Ala Asn
   225                 230                 235                 240
   Asn Val Asp Leu Asn Arg Asn Phe Lys Asp Asp Val Ala Gly Asp His
                   245                 250                 255
   Pro Asp Gly Lys Pro Trp Gln Pro Glu Ala Thr Ala Phe Met Asp Leu
               260                 265                 270
   Glu Gly Asn Thr Ser Phe Val Leu Gly Ala Asn Ile His Gly Gly Thr
           275                 280                 285
   Glu Val Val Asn Tyr Pro Trp Asp Asn Lys Lys Glu Arg His Ala Asp
       290                 295                 300
   Asp Glu Trp Tyr Lys Leu Ile Ser Arg Asn Tyr Ala Ala Ala Cys Gln
   305                 310                 315                 320
   Ser Ile Ser Ala Ser Tyr Met Thr Ser Glu Thr Asn Ser Gly Ile Ile
                   325                 330                 335
   Asn Gly Ser Asp Trp Tyr Val Ile Arg Gly Ser Arg Gln Asp Asn Ala
               340                 345                 350
   Asn Tyr Phe His Arg Leu Arg Glu Ile Thr Leu Glu Ile Ser Asn Thr
           355                 360                 365
   Lys Leu Val Pro Ala Ser Gln Leu Pro Lys Tyr Trp Asn Leu Asn Lys
       370                 375                 380
   Glu Ser Leu Leu Ala Leu Ile Glu Glu Ser Leu Tyr Gly Ile His Gly
   385                 390                 395                 400
   Thr Val Thr Ser Ala Ala Asn Gly Gln Pro Leu Lys Cys Gln Ile Leu
                   405                 410                 415
   Ile Glu Asn His Asp Lys Arg Asn Ser Asp Val Tyr Ser Asp Ala Thr
               420                 425                 430
   Thr Gly Tyr Tyr Val Arg Pro Ile Lys Ala Gly Thr Tyr Thr Val Lys
           435                 440                 445
   Tyr Lys Ala Glu Gly Tyr Pro Glu Ala Thr Arg Thr Ile Thr Ile Lys
       450                 455                 460
   Asp Lys Glu Thr Val Ile Met Asp Ile Ala Leu Gly Asn Ser Val Pro
   465                 470                 475                 480
   Leu Pro Val Pro Asp Phe Thr Ala Ser Pro Met Thr Ile Ser Val Gly
                   485                 490                 495
   Glu Ser Val Gln Phe Gln Asp Gln Thr Thr Asn Asn Pro Thr Asn Trp
               500                 505                 510
   Glu Trp Thr Phe Glu Gly Gly Gln Pro Ala Met Ser Thr Glu Gln Asn
           515                 520                 525
   Pro Leu Val Ser Tyr Ser His Pro Gly Gln Tyr Asp Val Thr Leu Lys
       530                 535                 540
   Val Trp Asn Ala Ser Gly Ser Asn Thr Ile Thr Lys Glu Lys Phe Ile
   545                 550                 555                 560
   Thr Val Asn Ala Val Met Pro Val Ala Glu Phe Val Gly Thr Pro Thr
                   565                 570                 575
   Glu Ile Glu Glu Gly Gln Thr Val Ser Phe Gln Asn Gln Ser Thr Asn
               580                 585                 590
   Ala Thr Asn Tyr Val Trp Ile Phe Asp Gly Gly Thr Pro Ala Thr Ser
           595                 600                 605
   Glu Asp Glu Asn Pro Thr Val Leu Tyr Ser Lys Ala Gly Gln Tyr Asp
       610                 615                 620
   Val Thr Leu Lys Ala Ile Ser Ala Ser Gly Glu Thr Val Lys Thr Lys
   625                 630                 635                 640
   Glu Lys Tyr Ile Thr Val Lys Lys Ala Pro Val Pro Ala Pro Val Ala
                   645                 650                 655
   Asp Phe Glu Gly Thr Pro Arg Lys Val Lys Lys Gly Glu Thr Val Thr
               660                 665                 670
   Phe Lys Asp Leu Ser Thr Asn Asn Pro Thr Ser Trp Leu Trp Val Phe
           675                 680                 685
   Glu Gly Gly Ser Pro Ala Thr Ser Thr Glu Gln Asn Pro Val Val Thr
       690                 695                 700
   Tyr Asn Glu Thr Gly Lys Tyr Asp Val Gln Leu Thr Ala Thr Asn Glu
   705                 710                 715                 720
   Gly Gly Ser Asn Val Lys Lys Ala Glu Asp Tyr Ile Glu Val Ile Leu
                   725                 730                 735
   Asp Asp Ser Val Glu Asp Ile Val Ala Gln Thr Gly Ile Val Ile Arg
               740                 745                 750
   Pro Gln Asn Gly Thr Lys Gln Ile Leu Ile Glu Ala Asn Ala Ala Ile
           755                 760                 765
   Lys Ala Ile Val Leu Tyr Asp Ile Asn Gly Arg Val Val Leu Lys Thr
       770                 775                 780
   Thr Pro Asn Gln Leu Arg Ser Thr Val Asp Leu Ser Ile Leu Pro Glu
   785                 790                 795                 800
   Gly Ile Tyr Thr Ile Asn Ile Lys Thr Glu Lys Ser Ala Arg Thr Glu
                   805                 810                 815
   Lys Ile His Ile Gly
               820
(2) INFORMATION FOR SEQ ID NO: 434
   (i) SEQUENCE CHARACTERISTICS:
      (A) LENGTH: 223 amino acids
      (B) TYPE: amino acid
      (D) TOPOLOGY: linear
   (ii) MOLECULE TYPE: protein
   (iii) HYPOTHETICAL: YES
   (vi) ORIGINAL SOURCE:
      (A) ORGANISM: Porphyromonas gingivalis
   (ix) FEATURE:
      (A) NAME/KEY: misc_feature
      (B) LOCATION 1...223
   (xi) SEQUENCE DESCRIPTION: SEQ ID NO: 434
   Met Lys Lys Ser Ser Val Val Ala Ser Val Leu Ala Val Ala Leu Val
   1               5                   10                  15
   Phe Ala Gly Cys Gly Leu Asn Asn Met Ala Lys Gly Gly Leu Ile Gly
               20                  25                  30
   Ala Gly Val Gly Gly Ala Ile Gly Ala Gly Val Gly Asn Val Ala Gly
           35                  40                  45
   Asn Thr Ala Val Gly Ala Ile Val Gly Thr Ala Val Gly Gly Ala Ala
       50                  55                  60
   Gly Ala Leu Ile Gly Lys Lys Met Asp Lys Gln Lys Lys Glu Leu Glu
   65                  70                  75                  80
   Ala Ala Val Pro Asp Ala Thr Ile Gln Thr Val Asn Asp Gly Glu Ala
                   85                  90                  95
   Ile Leu Val Thr Phe Asp Ser Gly Ile Leu Phe Ala Thr Asn Ser Ser
               100                 105                 110
   Thr Leu Ser Pro Asn Ser Arg Thr Ala Leu Thr Lys Phe Ala Ala Asn
           115                 120                 125
   Met Asn Lys Asn Pro Asp Thr Asp Ile Arg Ile Val Gly His Thr Asp
       130                 135                 140
   Asn Thr Gly Ser Asp Lys Ile Asn Asp Pro Leu Ser Glu Arg Arg Ala
   145                 150                 155                 160
   Ala Ser Val Tyr Ser Phe Leu Asn Ser Gln Gly Val Ser Met Ser Arg
                   165                 170                 175
   Met Ala Ala Glu Gly Arg Gly Ser His Glu Pro Val Ala Asp Asn Ser
               180                 185                 190
   Thr Val Ala Gly Arg Ser Ala Asn Arg Arg Val Glu Val Tyr Ile Leu
           195                 200                 205
   Pro Asn Ala Lys Met Ile Glu Gln Ala Gln Gln Gly Thr Leu Lys
       210                 215                 220
(2) INFORMATION FOR SEQ ID NO: 435
   (i) SEQUENCE CHARACTERISTICS:
      (A) LENGTH: 337 amino acids
      (B) TYPE: amino acid
      (D) TOPOLOGY: linear (ii) MOLECULE TYPE: protein (iii) HYPOTHETICAL: YES
   (vi) ORIGINAL SOURCE:
      (A) ORGANISM: Porphyromonas gingivalis
   (ix) FEATURE:
      (A) NAME/KEY: misc_feature
      (B) LOCATION 1...337
   (xi) SEQUENCE DESCRIPTION: SEQ ID NO: 435
   Met Ser Lys Lys Ser Ile Leu Leu Leu Cys Cys Ser Leu Cys Phe Ile
   1               5                   10                  15
   Ser Ala Thr Lys Ala Val Thr Pro Val Arg Asn Val Arg Asn Ser Gln
               20                  25                  30
   Val Asn Ser Lys Ala Lys Thr Glu Arg Thr Lys Pro Ser Asp Ser Val
           35                  40                  45
   Arg Tyr Ile Ser Asn Met Ile Ala Asp Arg Leu Glu Phe Arg Asn Lys
       50                  55                  60
   Ile Ser Ser Glu Lys Glu Val Arg Lys Ala Glu Tyr Glu Asn Arg Leu
   65                  70                  75                  80
   Ala Met Glu Ala Leu Asn Tyr Pro Ala Ile Asp Leu Tyr Gly Glu Asp
                   85                  90                  95
   Ser Trp Ser Glu Tyr Val Asn Pro Phe Val Gly Ala Gly Thr Asp Val
               100                 105                 110
   Glu Ile Pro Asn Ser Tyr Asp Ile Asp Cys Ser Ser Phe Val Met Pro
           115                 120                 125
   Val Glu Asp Lys Gln Val Thr Ser Gln Phe Gly Tyr Arg Arg Arg Phe
       130                 135                 140
   Gly Arg Met His Tyr Gly Ile Asp Leu Ser Val Asn Arg Gly Asp Thr
   145                 150                 155                 160
   Ile Arg Ala Ala Phe Asp Gly Lys Val Arg Val Arg Ser Tyr Glu Ala
                   165                 170                 175
   Arg Gly Tyr Gly Tyr Tyr Ile Val Leu Arg His Pro Asn Gly Leu Glu
               180                 185                 190
   Thr Val Tyr Gly His Met Ser Arg Gln Leu Val Asp Glu Asn Gln Ile
           195                 200                 205
   Val Arg Ala Gly Gln Pro Ile Gly Leu Gly Gly Ser Thr Gly Arg Ser
       210                 215                 220
   Thr Gly Pro His Leu His Phe Glu Thr Arg Phe Met Gly Ile Pro Ile
   225                 230                 235                 240
   Asn Pro Ser Thr Ile Ile Asp Phe Asp Asn Gly Val Pro Leu Arg Asp
                   245                 250                 255
   Ile Tyr Thr Phe Lys Arg Gly Ser Asn Ser Arg Tyr Ala Lys Ala Ser
               260                 265                 270
   Lys Thr Ser Ser Arg Tyr Ala Lys Lys Gly Lys Lys Gly Arg Gln Ala
           275                 280                 285
   Ser Ser Pro Met Thr Tyr Arg Ile Lys Lys Gly Asp Thr Leu Glu Thr
       290                 295                 300
   Ile Ala Lys Arg His Gly Thr Ser Val Gln Lys Leu Cys Ala Thr Asn
   305                 310                 315                 320
   Gly Ile Gly Lys Ser Lys Ile Leu Thr Pro Gly Lys Ala Leu Arg Ile
                   325                 330                 335
   Lys
(2) INFORMATION FOR SEQ ID NO: 525
   (i) SEQUENCE CHARACTERISTICS:
      (A) LENGTH: 556 amino acids
      (B) TYPE: amino acid
      (D) TOPOLOGY: linear (ii) MOLECULE TYPE: protein (iii) HYPOTHETICAL: YES
   (vi) ORIGINAL SOURCE:
      (A) ORGANISM: Porphyromonas gingivalis
   (ix) FEATURE:
      (A) NAME/KEY: misc_feature
      (B) LOCATION 1...556
   (xi) SEQUENCE DESCRIPTION: SEQ ID NO: 525
   Met Lys Lys Leu Leu Gln Ala Lys Ala Leu Ile Leu Ala Leu Gly Leu
   1               5                   10                  15
   Phe Gln Leu Pro Ala Ile Ala Gln Thr Gln Met Gln Ala Asp Arg Thr
               20                  25                  30
   Asn Gly Gln Phe Ala Thr Glu Glu Met Gln Arg Ala Phe Gln Glu Thr
           35                  40                  45
   Asn Pro Pro Ala Gly Pro Val Arg Ala Ile Ala Glu Tyr Glu Arg Ser
       50                  55                  60
   Ala Ala Val Leu Val Arg Tyr Pro Phe Gly Ile Pro Met Glu Leu Ile
   65                  70                  75                  80
   Lys Glu Leu Ala Lys Asn Asp Lys Val Ile Thr Ile Val Ala Ser Glu
                   85                  90                  95
   Ser Gln Lys Asn Thr Val Ile Thr Gln Tyr Thr Gln Ser Gly Val Asn
               100                 105                 110
   Leu Ser Asn Cys Asp Phe Ile Ile Ala Lys Thr Asp Ser Tyr Trp Thr
           115                 120                 125
   Arg Asp Tyr Thr Gly Trp Phe Ala Met Tyr Asp Thr Asn Lys Val Gly
       130                 135                 140
   Leu Val Asp Phe Ile Tyr Asn Arg Pro Arg Pro Asn Asp Asp Glu Phe
   145                 150                 155                 160
   Pro Lys Tyr Glu Ala Gln Tyr Leu Gly Ile Glu Met Phe Gly Met Lys
                   165                 170                 175
   Leu Lys Gln Thr Gly Gly Asn Tyr Met Thr Asp Gly Tyr Gly Ser Ala
               180                 185                 190
   Val Gln Ser His Ile Ala Tyr Thr Glu Asn Ser Ser Leu Ser Gln Ala
           195                 200                 205
   Gln Val Asn Gln Lys Met Lys Asp Tyr Leu Gly Ile Thr His His Asp
       210                 215                 220
   Val Val Gln Asp Pro Asn Gly Glu Tyr Ile Asn His Val Asp Cys Trp
   225                 230                 235                 240
   Gly Lys Tyr Leu Ala Pro Asn Lys Ile Leu Ile Arg Lys Val Pro Asp
                   245                 250                 255
   Asn His Pro Gln His Gln Ala Leu Glu Asp Met Ala Ala Tyr Phe Ala
               260                 265                 270
   Ala Gln Thr Cys Ala Trp Gly Thr Lys Tyr Glu Val Tyr Arg Ala Leu
           275                 280                 285
   Ala Thr Asn Glu Gln Pro Tyr Thr Asn Ser Leu Ile Leu Asn Asn Arg
       290                 295                 300
   Val Phe Val Pro Val Asn Gly Pro Ala Ser Val Asp Asn Asp Ala Leu
   305                 310                 315                 320
   Asn Val Tyr Lys Thr Ala Met Pro Gly Tyr Glu Ile Ile Gly Val Lys
                   325                 330                 335
   Gly Ala Ser Gly Thr Pro Trp Leu Gly Thr Asp Ala Leu His Cys Arg
               340                 345                 350
   Thr His Glu Val Ala Asp Lys Gly Tyr Leu Tyr Ile Lys His Tyr Pro
           355                 360                 365
   Ile Leu Gly Glu Gln Ala Gly Pro Asp Tyr Lys Ile Glu Ala Asp Val
       370                 375                 380
   Val Ser Cys Ala Asn Ala Thr Ile Ser Pro Val Gln Cys Tyr Tyr Arg
   385                 390                 395                 400
   Ile Asn Gly Ser Gly Ser Phe Lys Ala Ala Asp Met Thr Met Glu Ser
                   405                 410                 415
   Thr Gly His Tyr Thr Tyr Ser Phe Thr Gly Leu Asn Lys Asn Asp Lys
               420                 425                 430
   Val Glu Tyr Tyr Ile Ser Ala Ala Asp Asn Ser Gly Arg Lys Glu Thr
           435                 440                 445
   Tyr Pro Phe Ile Gly Glu Pro Asp Pro Phe Lys Phe Thr Cys Met Asn
       450                 455                 460
   Glu Thr Asn Thr Cys Thr Val Thr Gly Ala Ala Lys Ala Leu Arg Ala
   465                 470                 475                 480
   Trp Phe Asn Ala Gly Arg Ser Glu Leu Ala Val Ser Val Ser Leu Asn
                   485                 490                 495
   Ile Ala Gly Thr Tyr Arg Ile Lys Leu Tyr Asn Thr Ala Gly Glu Glu
               500                 505                 510
   Val Ala Ala Met Thr Lys Glu Leu Val Ala Gly Thr Ser Val Phe Ser
           515                 520                 525
   Met Asp Val Tyr Ser Gln Ala Pro Gly Thr Tyr Val Leu Val Val Glu
       530                 535                 540
   Gly Asn Gly Ile Arg Glu Thr Met Lys Ile Leu Lys
   545                 550                 555
(2) INFORMATION FOR SEQ ID NO: 526
   (i) SEQUENCE CHARACTERISTICS:
      (A) LENGTH: 428 amino acids
      (B) TYPE: amino acid
      (D) TOPOLOGY: linear (ii) MOLECULE TYPE: protein (iii) HYPOTHETICAL: YES
   (vi) ORIGINAL SOURCE:
      (A) ORGANISM: Porphyromonas gingivalis
   (ix) FEATURE:
      (A) NAME/KEY: misc_feature
      (B) LOCATION 1...428
   (xi) SEQUENCE DESCRIPTION: SEQ ID NO: 526
   Met Lys Leu Ser Ser Lys Lys Ile Leu Ala Ile Ile Ala Leu Leu Thr
   1               5                   10                  15
   Met Gly His Ala Val Gln Ala Gln Phe Val Pro Ala Pro Thr Thr Gly
               20                  25                  30
   Ile Arg Met Ser Val Thr Thr Thr Lys Ala Val Gly Glu Lys Ile Glu
           35                  40                  45
   Leu Leu Val His Ser Ile Glu Lys Lys Gly Ile Trp Ile Asp Leu Asn
       50                  55                  60
   Gly Asp Ala Thr Tyr Gln Gln Gly Glu Glu Ile Thr Val Phe Asp Glu
   65                  70                  75                  80
   Ala Tyr His Glu Tyr Thr Ile Gly Thr Gln Thr Leu Thr Ile Tyr Gly
                   85                  90                  95
   Asn Thr Thr Arg Leu Gly Cys Arg Ser Thr Gly Ala Thr Ala Val Asp
               100                 105                 110
   Val Thr Lys Asn Pro Asn Leu Thr Tyr Leu Ala Cys Pro Lys Asn Asn
           115                 120                 125
   Leu Lys Ser Leu Asp Leu Thr Gln Asn Pro Lys Leu Leu Arg Val Trp
       130                 135                 140
   Cys Asp Ser Asn Glu Ile Glu Ser Leu Asp Leu Ser Gly Asn Pro Ala
   145                 150                 155                 160
   Leu Ile Ile Leu Gly Cys Asp Arg Asn Lys Leu Thr Glu Leu Lys Thr
                   165                 170                 175
   Asp Asn Asn Pro Lys Leu Ala Ser Leu Trp Cys Ser Asp Asn Asn Leu
               180                 185                 190
   Thr Glu Leu Glu Leu Ser Ala Asn Pro Arg Leu Asn Asp Leu Trp Cys
           195                 200                 205
   Phe Gly Asn Arg Ile Thr Lys Leu Asp Leu Ser Ala Asn Pro Leu Leu
       210                 215                 220
   Val Thr Leu Trp Cys Ser Asp Asn Glu Leu Ser Thr Leu Asp Leu Ser
   225                 230                 235                 240
   Lys Asn Ser Asp Val Ala Tyr Leu Trp Cys Ser Ser Asn Lys Leu Thr
                   245                 250                 255
   Ser Leu Asn Leu Ser Gly Val Lys Gly Leu Ser Val Leu Val Cys His
               260                 265                 270
   Ser Asn Gln Ile Ala Gly Glu Glu Met Thr Lys Val Val Asn Ala Leu
           275                 280                 285
   Pro Thr Leu Ser Pro Gly Ala Gly Ala Gln Ser Lys Phe Val Val Val
       290                 295                 300
   Asp Leu Lys Asp Thr Asp Glu Lys Asn Ile Cys Thr Val Lys Asp Val
   305                 310                 315                 320
   Glu Lys Ala Lys Ser Lys Asn Trp Arg Val Phe Asp Phe Asn Gly Asp
                   325                 330                 335
   Ser Asp Asn Met Leu Pro Tyr Glu Gly Ser Pro Thr Ser Asn Leu Ala
               340                 345                 350
   Val Asp Ala Pro Thr Val Arg Ile Tyr Pro Asn Pro Val Gly Arg Tyr
           355                 360                 365
   Ala Leu Val Glu Ile Pro Glu Ser Leu Leu Gly Gln Glu Ala Ala Leu
       370                 375                 380
   Tyr Asp Met Asn Gly Val Lys Val Tyr Ser Phe Ala Val Glu Ser Leu
   385                 390                 395                 400
   Arg Gln Asn Ile Asp Leu Thr His Leu Pro Asp Gly Thr Tyr Phe Phe
                   405                 410                 415
   Arg Leu Asp Asn Tyr Thr Thr Lys Leu Ile Lys Gln
               420                 425

## Description

### FIELD OF THE INVENTION

The present invention relates to *P. gingivalis* nucleotide sequences, *P. gingivalis* polypeptides and probes for detection of *P*. *gingivalis*. The *P. gingivalis* polypeptides and nucleotides can be used in compositions for use in raising an immune response in a subject against *P. gingivalis* and treating or preventing or reducing the severity of the condition known as periodontitis.

### BACKGROUND OF THE INVENTION

Periodontal diseases are bacterial-associated inflammatory diseases of the supporting tissues of the teeth and range from the relatively mild form of gingivitis, the non-specific, reversible inflammation of gingival tissue to the more aggressive forms of periodontitis which are characterised by the destruction of the tooth's supporting structures. Periodontitis is associated with a subgingival infection of a consortium of specific Gram-negative bacteria that leads to the destruction of the periodontium and is a major public health problem. One bacterium that has attracted considerable interest is *P. gingivalis* as the recovery of this microorganism from adult periodontitis lesions can be up to 50% of the subgingival anaerobically cultivable flora, whereas *P. gingivalis* is rarely recovered, and then in low numbers, from healthy sites. A proportional increase in the level of *P. gingivalis* in subgingival plaque has been associated with an increased severity of periodontitis and eradication of the microorganism from the cultivable subgingival microbial population is accompanied by resolution of the disease. The progression of periodontitis lesions in non-human primates has been demonstrated with the subgingival implantation of *P. gingivalis*. These findings in both animals and humans suggest a major role for *P. gingivalis* in the development of adult periodontitis.

*P. gingivalis* is a black-pigmented, anaerobic, asaccharolytic, proteolytic Gram-negative rod that obtains energy from the metabolism of specific amino acids. The microorganism has an absolute growth requirement for iron, preferentially in the form of haeme or its Fe(III)

In order to develop an efficacious and safe vaccine to prevent, eliminate or reduce *P. gingiva*/*is* colonisation it is necessary to identify and produce antigens that are involved in virulence that have utility as immunogens possibly through the generation of specific antibodies. Whilst it is possible to attempt to isolate antigens directly from cultures of *P. gingiva*/*is* this is often difficult. For example as mentioned above, *P. gingivalis* is a strict anaerobe and can be difficult to isolate and grow. It is also known that, for a number of organisms, when cultured *in vitro* that many virulence genes are down regulated and the encoded proteins are no longer expressed. If conventional chemistry techniques were applied to purify vaccine candidates potentially important (protective) molecules may not be identified. With DNA sequencing, as the gene is present (but not transcribed) even when the organism is grown *in vitro* it can be identified, cloned and produced as a recombinant DNA protein. Similarly, a protective antigen or therapeutic target may be transiently expressed by the organism *in vitro* or produced in low levels making the identification of these molecules extremely difficult by conventional methods.

With serological identification of therapeutic targets one is limited to those responses which are detectable using standard methods such as Western Blotting or ELISA. The limitation here is the both the level of response that is generated by the animal or human and determining whether this response is protective, damaging or irrelevant. No such limitation is present with a sequencing approach to the identification of potential therapeutic or prophylactic targets.

It is also well known that *P. gingivalis* produces a range of broadly active proteases (University of Melbourne, International Patent Application No WO97/16542, US Patent Nos 5,475,097 and 5,523,390), which make the identification of intact proteins difficult because of their degradation by these proteases.

### SUMMARY OF THE INVENTION

The present inventors have attempted to isolate *P. gingivalis* nucleotide sequences which can be used for recombinant production of *P. gingivalis* polypeptides and to develop nucleotide probes specific for *P. gingivalis*. The DNA sequences listed below have been selected from a large number of *P. gingivalis* sequences according to their indicative potential as vaccine candidates. This intuitive step involved comparison of the deduced protein sequence from the *P. gingivalis* DNA sequences to the known protein sequence databases. Some of the characteristics used to select useful vaccine candidates include; the expected cellular location, such as outer membrane proteins or secreted proteins, particular functional activities of similar proteins such as those with an enzymatic or proteolytic activity, proteins involved in essential metabolic pathways that when inactivated or blocked may be deleterious or lethal to the organism, proteins that might be expected to play a role in the pathogenesis of the organism eg. red cell lysis, cell agglutination or cell receptors and proteins which are paralogues to proteins with proven vaccine efficacy.

In a first aspect the present invention provides an isolated antigenic *Porphorymonas gingivalis* polypeptide, the polypeptide comprising;
(i) the amino acid sequence of SEQ ID NO. 426;
(ii) the amino acid sequence of residues 24 to 821 or 27 to 821 of SEQ ID NO. 426; or
(iii) an amino acid sequence at least 85% identical to an amino acid sequence of (i).

In an embodiment of the present invention, the polypeptide comprises an amino acid sequence at least 95% identical to the amino acid sequence of (i).

As used herein % identity for polypeptides is to be calculated using the alignment algorithm of Needleman and Munsch (9) using a standard protein scoring matrix (Blosum 50).

In a preferred embodiment of the present invention the polypeptide comprises an amino acid sequence selected from the group consisting of SEQ. ID. NO. 426 or SEQ ID NO. 301.

In a second aspect the present invention provides an isolated DNA molecule, the DNA molecule comprising a nucleotide sequence which encodes the polypeptide of the first aspect of the present invention.

It is preferred that the isolated DNA molecule comprises the nucleotide sequence of SEQ. ID. NO. 162 or SEQ ID NO. 37.

In a third aspect, the present invention provides a recombinant expression vector comprising the DNA molecule of the second aspect of the present invention operably linked to a transcription regulatory element.

The present invention also provides an isolated cell comprising this recombinant expression vector.

In a further aspect, the present invention provides a method for producing a *P*. *gingiva*/*is* polypeptide comprising culturing the cell under conditions that permit expression of the polypeptide.

In yet a further aspect, the present invention provides a pharmaceutical composition comprising (i) an effective amount of (a) at least one polypeptide of the first aspect of the present invention, (b) at least one DNA molecule of the second aspect of the present invention, and/or (c) a polypeptide of at least 40 amino acids having a contiguous sequence of at least 40 amino acids identical to a contiguous amino acid sequence of SEQ ID NO. 426, wherein the polypeptide is capable of raising an immune response against *P. gingivalis*, and (ii) a pharmaceutically acceptable carrier. It is preferred that the pharmaceutically acceptable carrier is an adjuvant. In other aspects, the present invention provides such a composition for use in raising an immune response directed to *P. gingivalis* in a subject, or for treating *P. gingivalis* infection. The treatment may be prophylactic or therapeutic.

In yet another aspect, the present invention provides an antibody raised against a polypeptide of the first aspect of the invention. The antibody may be polyclonal or monoclonal. The present invention also provides compositions including these antibodies. It is preferred that these compositions are adapted for oral use and may be, for example, dentrifices, mouthwashes, etc.

The present invention also provides a method for detecting the presence of a DNA molecule of the second aspect of the inventionin a sample comprising:
(a) contacting a sample with a nucleotide probe comprising at least 18 nucleotides and having a contiguous sequence of at least 18 nucleotides identical to a contiguous nucleotide sequence selected from the group consisting of SEQ. ID. NO. 37 under conditions in which a hybrid can form between the probe and the DNA molecule of the second aspect of the invention in the sample; and
(b) detecting the hybrid formed in step (a), wherein detection of a hybrid indicates the presence of the DNA molecule of the second aspect of the invention in the sample.

### DETAILED DESCRIPTION

### Definitions

A purified or isolated polypeptide or a substantially pure preparation of a polypeptide are used interchangeably herein and, as used herein, mean a polypeptide that has been separated from other proteins, lipids, and nucleic acids with which it naturally occurs. Preferably, the polypeptide is also separated from substances, e.g., antibodies or gel matrix, e.g., polyacrylamide, which are used to purify it. Preferably, the polypeptide constitutes at least 10, 20, 50 70, 80 or 95% dry weight of the purified preparation. Preferably, the preparation contains: sufficient polypeptide to allow protein sequencing; at least 1, 10, or 100 mg of the polypeptide.

A purified preparation of cells refers to, in the case of plant or animal cells, an in vitro preparation of cells and not an entire intact plant or animal. In the case of cultured cells or microbial cells, it consists of a preparation of at least 10% and more preferably 50% of the subject cells.

A purified or isolated or a substantially pure nucleic acid, e.g., a substantially pure DNA, (are terms used interchangeably herein) is a nucleic acid which is one or both of the following: not immediately contiguous with both of the coding sequences with which it is immediately contiguous (i.e., one at the 5' end and one at the 3' end) in the naturally occurring genome of the organism from which the nucleic acid is derived; or which is substantially free of a nucleic acid with which it occurs in the organism from which the nucleic acid is derived. The term includes, for example, a recombinant DNA which is incorporated into a vector, e.g., into an autonomously replicating plasmid or virus, or into the genomic DNA of a prokaryote or eukaryote, or which exists as a separate molecule (e.g., a cDNA or a genomic DNA fragment produced by PCR or restriction endonuclease treatment) independent of other DNA sequences. Substantially pure DNA also includes a recombinant DNA which is part of a hybrid gene encoding additional *P. gingivalis* DNA sequence.

A "contig" as used herein is a nucleic acid representing a continuous stretch of genomic sequence of an organism.

An "open reading frame", also referred to herein as ORF, is a region of nucleic acid which encodes a polypeptide. This region may represent a portion of a coding sequence or a total sequence and can be determined from a stop to stop codon or from a start to stop codon.

As used herein, a "coding sequence" is a nucleic acid which is transcribed into messenger RNA and/or translated into a polypeptide when placed under the control of appropriate regulatory sequences. The boundaries of the coding sequence are determined by a translation start codon at the five prime terminus and a translation stop code at the three prime terminus. A coding sequence can include but is not limited to messenger RNA synthetic DNA, and recombinant nucleic acid sequences.

A "complement" of a nucleic acid as used herein refers to an antiparallel or antisense sequence that participates in Watson-Crick base-pairing with the original sequence.

A "gene product" is a protein or structural RNA which is specifically encoded by a gene.

As used herein, the term "probe" refers to a nucleic acid, peptide or other chemical entity which specifically binds to a molecule of interest. Probes are often associated with or capable of associating with a label. A label is a chemical moiety capable of detection. Typical labels comprise dyes, radioisotopes, luminescent and chemiluminescent moieties, fluorophores, enzymes, precipitating agents, amplification sequences, and the like. Similarly, a nucleic acid, peptide or other chemical entity which specifically binds to a molecule of interest and immobilizes such molecule is referred herein as a "capture ligand". Capture ligands are typically associated with or capable of associating with a support such as nitro-cellulose, glass, nylon membranes, beads, particles and the like. The specificity of hybridization is dependent on conditions such as the base pair composition of the nucleotides, and the temperature and salt concentration of the reaction. These conditions are readily discernible to one of ordinary skill in the art using routine experimentation.

Homologous refers to the sequence similarity or sequence identity between two polypeptides or between two nucleic acid molecules. When a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided- by the number of positions compared x 100.

The terms peptides, proteins, and polypeptides are used interchangeably herein.

An "immunogenic component" as used herein is a moiety, such as an *P. gingivalis* polypeptide, analog or fragment thereof, that is capable of eliciting a humoral and/or cellular immune response in a host animal.

An "antigenic component" as used herein is a moiety, such as *P. gingivalis* polypeptide, analog or fragment thereof, that is capable of binding to a specific antibody with sufficiently high affinity to form a detectable antigen-antibody complex.

As used herein, the term "cell-specific promoter" means a DNA sequence that serves as a promoter, i.e., regulates expression of a selected DNA sequence operably linked to the promoter, and which effects expression of the selected DNA sequence in specific cells of a tissue. The term also covers so-called "leaky" promoters, which regulate expression of a selected DNA primarily in one tissue, but cause expression in other tissues as well.

As used herein, the term "control sequence" refers to a nucleic acid having a base sequence which is recognized by the host organism to effect the expression of encoded sequences to which they are ligated. The nature of such control sequences differs depending upon the host organism; in prokaryotes, such control sequences generally include a promoter, ribosomal binding site, terminators, and in some cases operators; in eukaryotes, generally such control sequences include promoters, terminators and in some instances, enhancers. The term control sequence is intended to include at a minimum, all components whose presence is necessary for expression, and may also include additional components whose presence is advantageous, for example, leader sequences.

As used herein, the term "operably linked" refers to sequences joined or ligated to function in their intended manner. For example, a control sequence is operably linked to coding sequence by ligation in such a way that expression of the coding sequence is achieved under conditions compatible with the control sequence and host cell.

A "sample" as used herein refers to a biological sample, such as, for example, tissue or fluid isolated from an individual (including without limitation plasma. serum, cerebrospinal fluid, lymph, tears, saliva and tissue sections) or from *in vitro* cell culture constituents, as well as samples from the environment.

The practice of the invention will employ, unless otherwise indicated, conventional techniques of chemistry, molecular biology, microbiology, recombinant DNA, and immunology well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (Editors), Current Protocols in Molecular Biology, Greene Pub. Associates and Wiley-Interscience (1988, including all updates until present).

### Pharmaceutically Acceptable Carriers

The antibodies, polypeptides and DNA of the present invention can be included in compositions which include a carrier or diluent. These compositions include pharmaceutical compositions where the carrier or diluent will be pharmaceutically acceptable. Pharmaceutically acceptable carriers or diluents include those used in compositions suitable for oral, rectal, nasal, topical (including buccal and sublingual), vaginal, parenteral (including subcutaneous, intramuscular, intravenous, intradermal, intrathecal and epidural) administration. They are non-toxic to recipients at the dosages and concentrations employed. Representative examples of pharmaceutically acceptable carriers or diluents include, but are not limited to; water, isotonic solutions which are preferably buffered at a physiological pH (such as phosphate-buffered saline or Tris-buffered saline) and can also contain one or more of, mannitol, lactose, trehalose, dextrose, glycerol, ethanol or polypeptides (such as human serum albumin). The compositions may conveniently be presented in unit dosage form and may be prepared by any of the methods well known in the art of pharmacy.

As will be well understood by those skilled in the art alterations may be made to the amino acid sequences set out in the Sequence Listings. These alterations may be deletions, insertions, or substitutions of amino acid residues. The altered polypeptides can be either naturally occurring (that is to say, purified or isolated from a natural source) or synthetic (for example, by performing site-directed metagenesis on the encoding DNA). It is intended that such altered polypeptides which have at least 85%, preferably at least 95% identity with the sequences set out in the Sequence Listing are within the scope of the present invention. Antibodies raised against these altered polypeptides will also bind to the polypeptides having one of the sequences set out in the Sequence Listings. The level of % identity is to be calculated as set out above.

Protein sequences are homologous if they are related by divergence from a common ancestor. Consequently, a species homologue of the protein will be the equivalent protein which occurs naturally in another species. Within any one species a homologue may exist as numerous allelic variants, and these will be considered homologues of the protein. Allelic variants and species homologues can be obtained by following standard techniques known to those skilled in the art.

An allelic variant will be a variant that is naturally occurring within an individual organism.

### Mutants, Variants and Homology - Nucleic Acids

Mutant polynucleotides will possess one or more mutations which are deletions, insertions, or substitutions of nucleotide residues. Mutants can be either naturally occurring (that is to say, isolated from a natural source) or synthetic (for example, by performing site-directed metagenesis on the DNA). It is thus apparent that polynucleotides of the invention can be either naturally occurring or recombinant (that is to say prepared using recombinant DNA techniques).

An allelic variant will be a variant that is naturally occurring within an individual organism.

Nucleotide sequences are homologous if they are related by divergence from a common ancestor. Consequently, a species homologue of the polynucleotide will be the equivalent polynucleotide which occurs naturally in another species. Within any one species a homologue may exist as numerous allelic variants, and these will be considered homologues of the polynucleotide. Allelic variants and species homologues can be obtained by following standard techniques known to those skilled in the art.

### Antibody Production

Antibodies, either polyclonal or monoclonal, which are specific for a polypeptide of the present invention can be produced by a person skilled in the art using standard techniques such as, but not limited to, those described by Harlow et al. Antibodies: A Laboratory Manual, Cold Springs Harbor Laboratory Press (1988), and D. Catty (editor), Antibodies: A Practical Approach, IRL Press (1988).

Various procedures known in the art may be used for the production of polyclonal antibodies to epitopes of a protein. For the production of polyclonal antibodies, a number of host animals are acceptable for the generation of antibodies by immunization with one or more injections of a polypeptide preparation, including but not limited to rabbits, mice, rats, etc. Various adjuvants may be used to increase the immunological response in the host animal, depending on the host species, including but not limited to Freund's (complete and incomplete), mineral gels such as aluminium hydroxide, surface active substances such as lysolecithin, pluronic polyols, polyanions, oil emulsions, keyhole lympet hemocyanins, dinitrophenol, and potentially useful human adjuvants such as BCG (bacille Calmette-Guerin) and Corynebacterium parvum.

A monoclonal antibody to an epitope of a protein may be prepared by using any technique which provides for the production of antibody molecules by continuous cell lines in culture. These include but are not limited to the hybridoma technique originally described by Kohler and Milstein (1975, Nature 256, 493-497), and the more recent human B-cell hybridoma technique (Kesber et al. 1983, Immunology Today 4:72) and EBV-hybridoma technique (Cole et al. 1985, Monoclonal Antibodies and Cancer Therapy, Alan R. Liss, Inc. pp. 77-96). In addition, techniques developed for the production of "chimeric antibodies" by splicing the genes from antibody molecule of appropriate antigen specificity together with genes from a human antibody molecule of appropriate biological activity may be used (Morrison et al. 1984, Proc. Natl. Acad. Sci., 81:6851-6855; Neuberger et al. 1984 Nature 312:604-608; Takeda et al. 1985 Nature 31:452-454). Alternatively, techniques described for the production of single chain antibodies (U.S. Patent 4,946,778) can be adapted to produce 4-specific single chain antibodies.

Recombinant human or humanized versions of monoclonal antibodies are a preferred embodiment for human therapeutic applications. Humanized antibodies may be prepared according to procedures in the literature (e.g. Jones et al. 1986, Nature 321:522-25; Reichman et al. 1988 Nature 332:323-27; Verhoeyen et al. 1988, Science 239:1534-36). The recently described "gene conversion metagenesis" strategy for the production of humanized monoclonal antibody may also be employed in the production of humanized antibodies (Carter et al. 1992 Proc. Natl. Acad. Sci. U.S.A. 89:4285-89). Alternatively, techniques for generating the recombinant phase library of random combinations of heavy and light regions may be used to prepare recombinant antibodies (e.g. Huse et al. 1989 Science 246:1275-81).

Antibody fragments which contain the idiotype of the molecule such as Fu F(ab1) and F(ab2) may be generated by known techniques. For example, such fragments include but are not limited to: the F(ab) E2 fragment which can be produced by pepsin digestion of the intact antibody molecule; the Fab' fragments which can be generated by reducing the disulfide bridges of the F(ab')2 fragment, and the two Fab fragments which can be generated by treating the antibody molecule with papain and a reducing agent. Alternatively, Fab expression libraries may be constructed (Huse et al. 1989, Science 246:1275-1281) to allow rapid and easy identification of monoclonal Fab fragment with the desired specificity to a protein.

### Adjuvants

"Adjuvant" means a composition comprised of one or more substances that enhances the immunogenicity and efficacy of a vaccine composition. Non-limiting examples of suitable adjuvants include squalane and squalene (or other oils of animal origin); block copolymers; detergents such as Tween®-80; Quail® A, mineral oils such as Drakeol® or Marcol®, vegetable oils such as peanut oil; Corynebacterium-derived adjuvants such as Corynebacterium parvum; Propionibacterium-derived adjuvants such as Propionibacterium acne; Mycobacterium bovis (Bacillus Calmetic and Guerinn or BCG); interleukins such as interleukin 2 and interleukin-12; monokines such as interleukin 1; tumour necrosis factor; interferons such as gamma interferon; combinations such as saponin-aluminium hydroxide or Quil-A aluminium hydroxide; liposomes; ISCOM adjuvant; mycobacterial cell wall extract; synthetic glycopeptides such as muramyl dipeptides or other derivatives; Avridine; Lipid A; dextran sulfate; DEAE-Dextran or DHAE-Dextran with aluminium phosphate; carboxypolymethylene such as. Carbopol' EMA®; acrylic copolymer emulsions such as Neocryl A640 (e.g. U.S. Pat. No. 5,047,238); vaccinia or animal posvirus proteins; sub-viral particle adjuvants such as cholera toxin, or mixtures thereof.

As used herein, stringent conditions are those that (1) employ low ionic strength and high temperature for washing, for example, 0.015 M NaCl/0.0015 M sodium citrate/0.1% NaDodSO4 at 50°C; (2) employ during hybridisation a denaturing agent such as formamide, for example, 50% (vol/vol) formamide with 0.1% bovine serum albumin, 0.1 % Ficoll, 0.1 % polyvinylpyrrolidone, 50 mM sodium phosphate buffer at pH 6.5 with 750 mM NaCl, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1 % SDS and 10% dextran sulfate at 42°C in 0.2 x SSC and 0.1 % SDS

As will be understood the present invention includes within its scope DNA vaccination. Further information regarding DNA vaccination may be found in Donnelly et al, Journal of Immunological Methods 176(1994) 145-152.

Throughout this specification the word "comprise", or variations such as "comprises" or "comprising", will be understood to imply the inclusion of a stated element or integer or group of elements or integers but not the exclusion of any other element or integer, or group of elements or integers.

### Preparation of the P. gingivalis library for sequencing.

To determine the DNA sequence of *P*. *gingivalis* genomic DNA was isolated from *P. gingiva*/*is* strain W50 (ATCC 53978) essentially by the method described by Mamur J. (J. Mol. Biol. 3, 208-218, 1961). Cloning of DNA fragments was performed essentially as described by Fleischmann et al., (Science; 269, 496-512, 1995)(2). Briefly, purified genomic DNA from *P. gingivalis* was nebulized to fragment the DNA and was treated with Bal31 nuclease to create blunt ends then run twice through preparative 1% agarose gels. DNA fragments of 1.6-2.0 kb were excised from the gel and the DNA recovered. This DNA was then ligated to the vector pUC18 (*Sma*I digested and dephosphorylated; Pharmacia) and electrophoresed through a 1% preparative agarose gel. The fragment comprising linear vector plus one insert was excised, purified and this process repeated to reduce any vector without insert contamination. The recovered vector plus insert DNA was blunt-ended with T4 DNA polymerase, then a final ligation to produce circular DNA was performed. Aliquots of Epicurian Coli Electroporation-Competent Cells (Stratagene) were transformed with the ligated DNA and plated out on SOB agar antibiotic diffusion plates containing X-gal and incubated at 37°C overnight. Colonies with inserts appeared white and those without inserts (vector alone) appeared blue. Plates were stored at 4°C until the white clones were picked and expanded for the extraction of plasmid DNA for sequencing.

### DNA sequencing

Plasmid DNA was prepared by picking bacterial colonies into 1.5ml of LB, TB or SOB broth supplemented with 50-100ug/ml Ampicillin in 96 deep well plates. Plasmid DNA was isolated using the QIAprep Spin or QIAprep 96 Turbo miniprep kits (QIAGEN GmbH, Germany). DNA was eluted into a 96 well gridded array and stored at -20C.

Sequencing reactions were performed using ABI PRISM Dye Terminator and ABI PRISM BIGDye Terminator Cycle Sequencing Ready Reaction kits with AmpliTaq DNA polymerase FS (PE Applied Biosystems, Foster City, CA) using the M 13 Universal forward and reverse sequencing primers. Sequence reactions were conducted on either a Perkin-Elmer GeneAmp 9700 (PE Applied Biosystems) or Hybaid PCR Express (Hybaid, UK) thermal cyclers. Sequencing reactions were analysed on ABI PRISM 377 DNA sequencers (PE Applied Biosystems).

The sequences obtained are set out below. It is to be noted that the present invention relates to PG21. The relationship between these sequences is set out in Table 1. The initiation codon was calculated using a combination of sequence homology alignment (FASTA), signal sequence prediction (PSORT, SignalP) or ORF prediction (GeneMark).

**Table 1: Reference table indicating the relationships of each sequence ID to the selected proteins.**

| **Protein name** | **DNA sequence of complete ORF** | **Amino acid sequence of complete ORF** | **DNA sequence of protein** | **Amino acid sequence of protein** |
|---|---|---|---|---|
| PG1 | 1 | 265 | 122 | 386 |
| | | | | |
| PG21 | 37 | 301 | 162 | 1426 |
| PG3 | 45 | 309 | 170 | 434 |
| PG30 | 46 | 310 | 171 | 435 |
| | | | | |
| PG96 | 118 | 382 | 261 | 525 |
| PG97 | 119 | 383 | 262 | 526 |

### DNA sequence analysis

DNA files in FASTA format were converted to GCG format files and imported into a database. The DNA files were translated into amino acid files using the program Flip obtained from ANGIS(Australian Genomic Information Service, University of Sydney, Australia). A series of bioinformatic analyses were performed on the proteins in order to select potential vaccine candidates. The programs used were FASTA homology searching (1), PSORT (2,3), SignalP (4), TopPred (5), and GeneMark (6). The proteins and their bioinformatic results were stored in the custom written database for search and retrieval of proteins with the desired characteristics

The FASTA homology results for these proteins were then examined for any alignment with a protein suggesting surface location or vaccine efficacy. All proteins were searched for homology against a non-redundant bacterial protein database compiled by ANGIS using the FASTA algorithm. The settings used for the FASTA searches were Ktup = 2, gap creation penalty = -12, gap extension penalty = -2, width for deriving alignment in opt = 16 and the Blosum 50 scoring matrix. Individual FASTA search results were examined for significant homology by statistical probability and amino acid alignments. The results are set out in Table 2.

Protein files were then trimmed to the first, second, third, fourth and fifth methionine residues using a protein trimming, program (ANGIS). The trimmed proteins were then subjected to PSORT analysis for the detection of signal sequences and the prediction of cell location. Proteins exhibiting a PSORT probability of outer membrane >0.8 were considered to indicate surface localisation. A second signal sequence detection program SignalP was also performed and, in certain instances, this program detected signals not identified with PSORT. All proteins identified by other methods were also analysed by PSORT and SignalP. Previously, the C-terminal amino acid of bacterial outer membrane proteins has been shown to be important for the assembly of the protein on the outer membrane (7). A typical structure definition for outer membrane proteins has been determined as the presence of a signal sequence at the N-terminus and a tyrosine or phenylalanine at the C-terminus. A number of the selected proteins exhibit this characteristic structure. The program TopPred was used to determine the presence and number of membrane spanning domains (MSDs) and the presence of such sequences indicates a preference to be attached to membranes such as the outer membrane. The results of PSORT, SignalP and TopPred analyses with the C-terminal amino acids of the selected proteins are set out in Table 3.

The 70 amino acids from the C-terminus of a number of *P. gingivalis* outer membrane proteins share 50-100% protein sequence identity. These proteins included RGP1, RGP2, KGP, HagA, HagC, HagD, prtH and prtT. This conserved motif may be involved in the attachment or sorting of proteins to the outer membrane. The protein data set was searched using FASTA homology as described above and a number of novel proteins were identified which demonstrate similar motifs at their C-termini. The results are listed in Table 4

**Table 2: FASTA protein homology results of complete ORFs against a non-redundant protein database.**

| **Protein name** | **Homology description** | **Genbank accession number** | **Length of homolog** | **Length of *P*. *gingivalis* protein** | **FASTA homology results** | | |
|---|---|---|---|---|---|---|---|
| | | | | | **Identity %** | **Overlap** | **E value** |
| **PG1** | 48kD outer membrane protein, Actinobacillus pleuropneumoniae | U24492 | 449aa | 451aa | 32 | 454aa | 1.40E-42 |
| **PG3** | Outer membrane porin F adhesin, Pseudomonas fluorescens | U19743 | 317aa | 223aa | 35 | 187aa | 1.10E-10 |
| | | | | | | | |
| **PG21** | surface antigen gene, Methanosarcina mazei | X84710 | 783aa | 821aa | 37 | 331aa | 6.20E-33 |
| | | | | | | | |
| **PG30** | Putative NlpD lipoprotein, Aquifex aeolicus | AE000754 | 187aa | 337aa | 42 | 142aa | 1.80E-12 |
| | | | | | | | |
| **PG75** | Class 3 outer membrane porin (porB), Neisseria meningitidis | U07191 | 332aa | 391aa | 23 | 239aa | 4.60E-01 |

**Table 3: Results of PSORT, SignalP and TopPred analysis of the proteins. The signal present column indicates the presence of a signal sequence detected with either PSORT or SignalP. The terms in parentheses indicates the type of signal sequence as determined by PSORT. The cell location & probability values are generated by PSORT and represent the probability of the protein being in the cell compartments outer membrane (OM), inner membrane (IM), periplasmic space (PC) or cytoplasm (C). The number of transmembrane domains (TMDs) was determined by TopPred and does not include uncleavable signal sequences.**

| **Protein name** | **Protein seqID number** | **Protein Length** | **Signal Present** | **Methionine in ORF** | **SignalP cleavage site** | **PSORT cleavage site** | **Cell Location & probability** | | | | **C-terminal Amino Acid** | **Number of TMD's** |
|---|---|---|---|---|---|---|---|---|---|---|---|---|
| | | | | | | | **OM** | **IM** | **PS** | **C** | | |
| PG1 | 386 | 451aa | Y | 1 | 14 | 34 | 0 | 0 | 0 | 0.22 | N | 0 |
| PG3 | 434 | 223aa | Y (lipoprotein) | 1 | - | 18 | 0.79 | 0.76 | 0 | 0 | K | 3 |
| | | | | | | | | | | | | |
| PG21 | 426 | 821aa | Y | 2 | 24 | 27 | 0.34 | 0 | 0.37 | 0 | G | 1 |
| | | | | | | | | | | | | |
| PG30 | 435 | 337aa | Y | 1 | 21 | 21 | 0.24 | 0 | 0.4 | 0 | K | 0 |
| | | | | | | | | | | | | |
| PG75 | 493 | 391aa | Y | 1 | 26 | 26 | 0.94 | 0 | 0.3 | 0 | H | 1 |
| | | | | | | | | | | | | |
| PG96 | 525 | 563aa | Y | 1 | 23 | 23 | 0.40 | 0 | 0.33 | 0 | K | 0 |
| PG97 | 526 | 437aa | Y | 1 | 23 | 23 | 0.32 | 0 | 0.65 | 0 | Q | 0 |

**Table 4: Percentage identity and percentage similarity of various proteins with the 70 amino acids from the C-terminal of the P. gingivalis arginine protease 1 (RGP1), arginine protease 2 (RGP2), and the cysteine protease/hemagglutinin (prtT).**

| **Protein name** | | **Percent identity** | | | | **Percent similarity** | | |
|---|---|---|---|---|---|---|---|---|
| | | **RGP1** | **RGP2** | **prtT** | | **RGP1** | **RGP2** | **prtT** |
| **PG21** | | 17 | 29 | 21 | | 40 | 57 | 49 |
| **PG96** | | 0 | 13 | 20 | | 0 | 24 | 43 |
| **PG97** | | 10 | 26 | 33 | | 14 | 47 | 61 |

### Cloning, expression and purification of recombinant P. gingivalis genes.

### PG1

Oligonucleotides to the 5' and 3' regions of the deduced protein were used to amplify the gene of interest from a preparation of *P. gingivalis* W50 genomic DNA using the TaqPlus Precision PCR System (Stratagene) and a PTC-100 (MJ Research) thermal cycler or similar device. The 5' oligonucleotide primer sequence was GCGCCATATGCTGGCCGAACCGGCC, the 3' oligonucleotide primer sequence was GCGCCTCGAGTCAATTCATTTCCTTATAGAG. The PCR fragment was purified, digested with Nde I, Xho I restriction enzymes (Promega) and ligated into the corresponding sites of the plasmid pProEx-1 (Gibco-BRL) and transformed into *E. coli* ER1793 cells (a gift from Elizabeth Raleigh, New England Biolabs). A resulting clone expressing the correct insert was selected and induced with or without 0.1mM IPTG (Promega) for expression of the recombinant protein. Expression of the recombinant protein was determined by SDS-PAGE analysis and Western Blot using the one of the rabbit antisera described above or an anti-hexahistidine antibody (Clontech) that detects the hexahistidine tag that was fused to *the P. gingivalis* recombinant protein. PG1 was purified by disruption of the *E. coli* cells by sonication in binding buffer (Novagen) and solubilisation by the addition of sarkosyl (N-Lauroyl sarcosine) to a 1% final concentration. There after the preparation was diluted to 0.1% sarkosyl in binding buffer, bound to a Nickel-nitrilotriacetic acid column (Ni-NTA; Qiagen), after washing bound proteins were eluted with 1M imidazole in elution buffer (Novagen) according to the Qiagen recommendations with 0.1% sarkosyl added to all buffers. Following purification samples were dialysed against 500mM NaCl, 20mM Tris, 0.1% sarkosyl at pH7.4 to remove the imidazole, concentrated as required and stored at 4°C until used. Purity and antigenicity were assessed by SDS-PAGE and Western blot using selected antisera (from those described above) and the protein concentration was determined by the BCA assay (Pierce).

### PG3

The methods used for PG3 were essentially the same as for PG1 with the following exceptions. The 5' oligonucleotide primer sequence was GCGCGTATACATGAAGAAATCAAGTGTAG, the 3' oligonucleotide primer sequence was GCGCAGATCTCTTCAGCGTACCTTGCTGTG and DNA was amplified with Pfu DNA polymerase (Stratagene). The PCR product was cloned directly into pCR-Blunt and transformed into *E. coli* Top10F'(In Vitrogen) before subcloning into the expression plasmid pGex-stop RBS(IV) using the Bst Z171 and Bgl II restriction sites and transformed into E. coli BL21DE3 (Pharmacia Biotech). The following modifications were made to the purification of PG3 from the PG1 method. Cells expressing the recombinant protein were disrupted by sonication in binding buffer and the insoluble inclusion bodies concentrated by centrifugation. Inclusion bodies were then solubilised in 6M urea (Sigma) in binding buffer and eluted with 6M urea added to the elution buffer. In some instances 6M guanidine hydrochloride (Sigma) was used instead of urea for these steps. Urea (or guanidine hydrochloride when it was substituted) was removed from the purified protein by sequential dialysis against reducing levels of urea (3M then 1.5M then 0.5M then 0M urea all in 50mM Tris, 500mM NaCl, 8% glycerol, pH7.4). Purified protein was stored frozen at -80°C until required. Protein concentration was determined by the Coomassie Plus protein assay (Pierce).

### PG30

The methods used for PG30 were essentially the same as for PG3 with the following exceptions. The predicted N-terminal signal sequence was removed from the recombinant protein. The 5' oligonucleotide primer sequence was TACGGAATTCGTGACCCCCGTCAGAAATGTGCGC, the 3' oligonucleotide primer sequence was CTATGCGGCCGCTTTGATCCTCAAGGCTTTGCCCGG and DNA was amplified with the Tth XL PCR kit. The PCR product was cloned into the expression plasmid pET24a using the Eco RI and Not I restriction sites and transformed into E. coli BL21DE3. Expression studies and immunoreactivity studies were carried out on whole E. coli lysates of PG30. 10ml cultures of recombinant E. coli were grown to an OD of 2.0 (A₆₀₀ₙₘ ) in terrific broth and the cells were induced with 0.5mM IPTG and samples taken for analysis at 4 hours post induction. Purification was not done for these studies.

### PG75

The methods used for PG75 were essentially the same as for PG30 with the following exceptions. The predicted N-terminal signal sequence was removed from the recombinant protein. The 5' oligonucleotide primer sequence was GGCGGGATCCGCTCAGGAGCAACTGAATGTGGTA, the 3' oligonucleotide primer sequence was GAGTGCGGCCGCTGTGGAACAAATTGCGCAATCCATC and DNA was amplified with the Tth XL PCR kit. The PCR product was cloned into the expression plasmid pET24a using the Bam HI and Not I restriction sites and transformed into *E. coli* BL21DE3. Expression studies and immunoreactivity studies were carried out on whole E. coli lysates. Purification was not done for these studies.

### PG96

The methods used for PG96 were essentially the same as for PG30 with the following exceptions. The predicted N-terminal signal sequence was removed from the recombinant protein. The 5' oligonucleotide primer sequence was TGCTGAGCTCCAAACGCAAATGCAAGCAGACCGA, the 3' oligonucleotide primer sequence was GAGTGCGGCCGCTTTTGAGAATTTTCATTGTCTCACG and DNA was amplified with the Tth XL PCR kit. The PCR product was cloned into the expression plasmid pET24a using the Sac I and Not I restriction sites and transformed into E. coli BL21DE3. Expression studies and immunoreactivity studies were carried out on whole E. coli lysates. Purification was not done for these studies.

### PG97

The methods used for PG97 were essentially the same as for PG30 with the following exceptions. The predicted N-terminal signal sequence was removed from the recombinant protein. The 5' oligonucleotide primer sequence was GGCGGGATCCCAGTTTGTTCCGGCTCCCACCACA, the 3' oligonucleotide primer sequence was GAGTGCGGCCGCTCTGTTTGATGAGCTTAGTGGTATA and DNA was amplified with the Tth XL PCR kit. The PCR product was cloned into the expression plasmid pET24a using the Bam HI and Not I restriction sites and transformed into E. coli BL21DE3. Expression studies and immunoreactivity studies were carried out on whole E. coli lysates. Purification was not done for these studies.

### Animal antisera and human patient sera.

Various antisera were raised for detecting the expression and refolding of the recombinant *P. gingivalis* proteins. A whole cell antisera was raised by injecting New Zealand White rabbits with 3 doses of sonicated *P. gingivalis* (strain W50) containing approximately 2mg of protein. The first dose was given in Freunds complete adjuvant (FCA) and the second and third doses were given in Freunds incomplete adjuvant (IFA) at 3 week intervals. Doses (1ml) were given intramuscularly into the hind legs and rabbits bled 7 days after the last dose, the blood clotted and serum removed and stored at -20°C until required. A second rabbit antisera was produced in a similar manner but using a sarkosyl insoluble fraction (each dose was 0.69mg of protein) derived from *P. gingivalis* W50 according to the method of Doidg and Trust T. *et al* 1994 as the immunogen. A third rabbit antisera was produced in a similar manner to the first only the sarkosyl soluble fraction (1mg of protein per dose) derived from *P. gingivalis* W50 cells according to the method of Doidg P. and Trust TJ. (1994 Infect Immun 62:4526-33) was used as the immunogen.

A "protected rat serum" pool was also used in these studies and was obtained from rats immunised with formalin killed whole *P. gingivalis* cells in FIA (strain ATCC 33277; 2 doses of 2x10⁹ cells, 3 weeks apart). Rats were then challenged 2 weeks after their last dose with live *P. gingiva*/*is* cells (strain 33277) given orally as previously described (Klaussen B. et al. 1991, Oral Microbiol Immunol 6:193-201) and the serum obtained from these rats 6 weeks after the final challenge inoculation at the time of sacrifice.

Human sera were obtained from adult patients undergoing treatment or assessment for periodontitis at an outpatient clinic. These patients had at least 6 teeth with 6mm attachment loss and had *P. gingivalis* present in their sub-gingival plaque as detected using a *P. gingivalis* specific DNA probe. Sera was pooled from these patients and compared to a pool of sera from periodontally healthy patients.

### Immunization and Murine Lesion Model Protocols

The mouse abscess model was used to assess the efficacy of immunising mice with recombinant *P. gingivalis* proteins in protecting mice from formation of a subcutaneous abscess. This model has been used by others as a predictor of potential vaccines against periodontal disease (Bird PS, et al. 1995 J. Periodontol. 66:351-362. BALB/c mice 6-8 weeks old were immunised by subcutaneously injecting them with 0.1 ml containing either 10 or 20µg of recombinant *P. gingivalis* protein, 20µg of *E*. *coli* lysate protein, 2 x 10⁹ formalin killed cells of *P. gingivalis* strain 33277 emulsified in incomplete Freund's adjuvant (IFA; Sigma) on day 0. At day 21 mice were reinjected with the same dose and then bled 1 week later and evaluated for antibody levels. At day 35 mice all mice were challenged with approximately 2 x 10⁹ cells of live *P. gingiva*/*is* (ATCC 33277) by subcutaneous injection in the abdomen. Following challenge mice were monitored daily for weight loss and the size of the lesion measured for the next 10 days. Lesion sizes were measured by length and width and expressed as mm². Groups were statistically analysed using a Kruskal-Wallis one-way ANOVA and were also individually examined using the unpaired t test or Mann-Whitney rank sum test using the Instat statistical package.

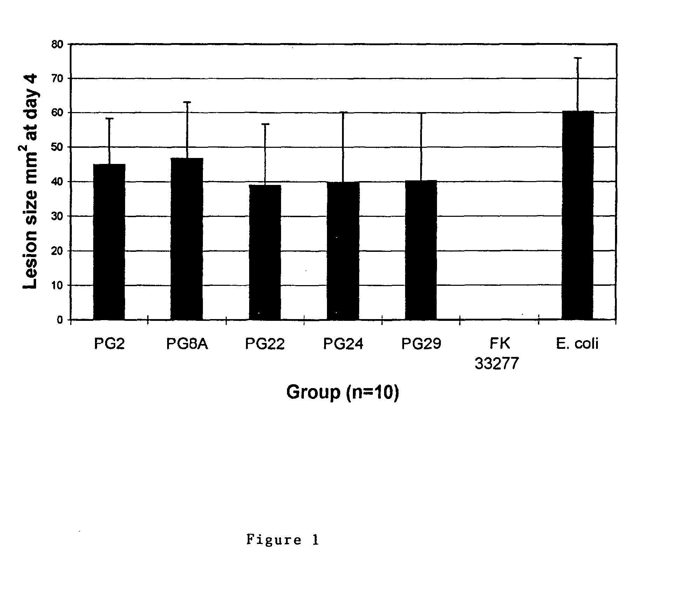

Figure 1 shows the results of one experiment at day 4 after challenge (lesions were at maximum size at this time point). Control mice immunised with *E. coli* lysate showed large lesions while mice immunised with killed cells of *P. gingiva*/*is* strain 33277 were fully protected. This indicates that whole cells provide protection against *P. gingiva*/*is* while *E. coli* protein immunised mice were not protected. Mice given the various PG recombinant proteins showed significant levels of protection for PG2, PG22, PG24 and PG29 (p<0.05 unpaired t test) while PG8A was not quite significantly different (p=0.07) compared to the *E. coli* control group.

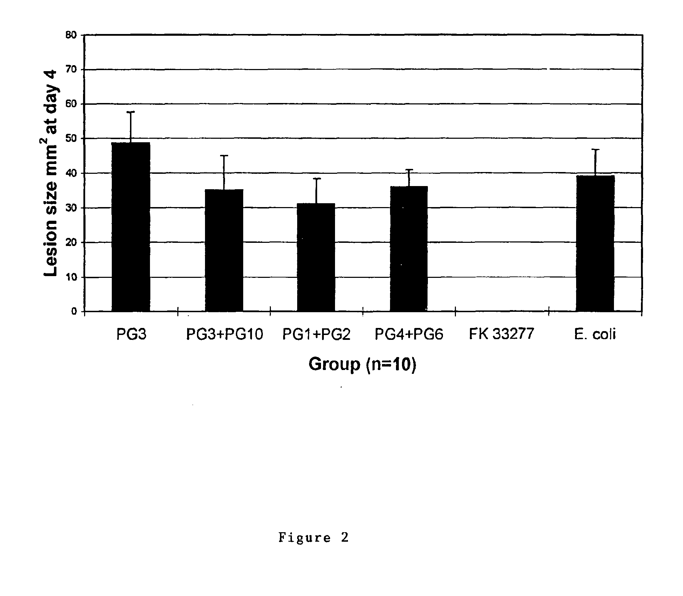

Figure 2 shows the results of a separate experiment using combinations of recombinant proteins. Mice given PG1 + PG2 showed a significant level of protection compared to control mice give *E. coli* lysate (p<0.026 unpaired t test).

### Immunoscreening

Cloned candidates were cultured in 15ml of Terrific broth, induced with IPTG and sampled at 4h post-induction. One ml of culture was removed, pelleted and the cells resuspended in a volume of PBS determined by dividing the OD A₆₀₀ₙₘ of the culture by 8. An aliquot of lysate (100µl) was added to 100µl of 2x sample reducing buffer (125mM Tris pH 6.8, 20% glycerol, 4% SDS, 80mM DTT, 0.03% bromophenol blue) and boiled for 10min. SDS-PAGE was performed according to the method of Laemmli UK. 1970 (Nature 227:680-685) using 4-20% 1.0mm Tris-Glycine gels (Novex) according to the manufacturers recommendations. Proteins were transferred onto Hybond-C Extra nitrocellulose membranes (Amersham) by transblotting and the membranes were then blocked for 2h at room temperature (RT) in 5% skim milk in 20mM Tris, 0.5M NaCl, 0.05% Tween-20, pH 7.5 (TTBS).

Immunoscreening was performed separately with the rabbit anti-*P*. *gingivalis* whole cell serum, the rat protective serum, a pool of human periodontal patients serum, and in many cases an anti-T7-Tag antibody HRP conjugate (Novagen). Prior to use, the rabbit, rat and human sera were diluted 1/5000, 1/1000 and 1/500 respectively in 5% skim milk in TTBS and absorbed with 100µl (for the rabbit serum) or 250µl (for the rat and human sera) *E*. *coli* extract (20mg/ml; Promega) for 6h at RT.

Membranes were incubated overnight at RT with the absorbed antisera, or for 1 hr at RT with 1/5000 diluted anti-T7-Tag conjugate. Following 3x10min washes with TTBS, HRP-conjugated anti-rabbit (Silenus), anti-mouse (Silenus) or anti-human (KPL) antibody, diluted 1/5000 in 5% skim milk in TTBS, was added for 1h at RT. Membranes were washed as before, prior to addition of TMB membrane peroxidase substrate (KPL) for detection of immunoreactive proteins. Results of reactivity for the recombinant *P. gingiva*/*is* proteins is shown in Table 5.

**Table 5: Immunoblot results of proteins expressed in E.coli against rabbit, rat and human antisera. Deduced MW was calculated from amino acid sequence of the P. gingiva/is proteins, some of which had their N-terminal signal sequences removed. Apparent MW was determined from SDS-PAGE gels. The N- and C-terminal tags add approximately 2.5 KDa to the deduced MW of the recombinant proteins. The symbols are + positive, - negative, +/- weak positive, ND not done.**

| **Protein number** | **Deduced MW (KDa)** | **Apparent MW (KDa)** | **Antisera reactivity** | | | |
|---|---|---|---|---|---|---|
| | | | **T7** | **Rabbit** | **Rat** | **Human** |
| PG1 | 47.5 | 63 | ND | - | - | - |
| PG3 | 22.6 | 18.3 | ND | -^{a} | - | - |
| | | | | | | |
| PG30 | 35.1 | 46.9 | + | - | - | - |
| | | | | | | |
| PG75 | 40.7 | 46.7 | + | - | - | - |
| | | | | | | |
| PG96 | 59.3 | 70.3 | + | + | + | + |
| PG97 | 44.4 | 57.5 | + | - | + | + |

| | | | | | | |
|---|---|---|---|---|---|---|
| **a.** Positive reaction detected with the rabbit antiserum to sarkosyl insoluble *P*. *gingivalis* antigen. **b.** Purified protein demonstrated weak positive reaction with the rabbit antiserum to whole *P. gingivalis*. | | | | | | |

### References.

1. Lipman DJ, Pearson WR. 1985. Rapid and sensitive protein similarity searches. Science 277:1435-1441.
2. Horton, P. and Nakai, K. (1996). A probabilistic classification system for predicting the cellular localization sites of proteins. Intellig. Syst. Mol. Biol. 4: 109-115.
3. Nakai K, Kanehisa M. 1991. Expert systems for predicting protein localization sites in Gram-negative bacteria. Proteins: Structure, Function, and Genetics 11:95-110.
4. Nielsen H, Engelbrecht J, Brunak S and von Heijne G. 1997. Identification of prokaryotic and eukaryotic signal peptides and prediction of their cleavage sites. Protein Engineering 10, 1-6.
5. Claros MG and G von Heijne. (1994). TopPred II: an improved software for membrane protein structure predictions. Comput. Appl. Biosci. 10: 685-686.
6. Borodovsky M, Rudd KE, and EV Koonin. (1994). Intrinsic and extrinsic approaches for detecting genes in a bacterial genome. Nucleic Acids Res. 22:4756-4767.
7. Struvye M, Moons M, Tommassen J. 1991. Carboxy-terminal phenylalanine is essential for the correct assembly of a bacterial outer membrane protein J. Mol. Biol. 218:141-148.
8. Aduse-Opoku J, Slaney JM, Rangarajan M, Muir J, Young KA, Curtis MA. 1997. The Tla receptor protein of Porphyromonas gingivalis W50: a homolog of the RI precursor (PrpRI) is an outer membrane receptor required for growth on low levels of hemin. J. Bacteriol. 179:4778-4788.
9. Needleman SB, Munsch CD. 1970. Ageneral method applicable to the search of similarity in the amino acid sequence of two proteins. J. Molec. Biol. 48: 443-453.

## Claims

1. An isolated antigenic *Porphyromonas gingivalis* polypeptide, the polypeptide comprising:
(i) the amino acid sequence of SEQ ID NO. 426;
(ii) the amino acid sequence of residues 24 to 821 or 27 to 821 of SEQ ID NO. 426; or
(iii) an amino acid sequence at least 85% identical to an amino acid sequence of(i).

2. A polypeptide as claimed in claim 1, comprising an amino acid sequence at least 95% identical to an amino acid sequence of (i).

3. A polypeptide as claimed in claim 1 or claim 2, wherein the amino acid sequence is SEQ ID NO. 426.

4. An isolated DNA molecule, the DNA molecule comprising a nucleotide sequence which encodes the polypeptide as claimed in claim 1, 2 or 3.

5. An isolated DNA molecule as claimed in claim 4 in which the DNA molecule comprises the nucleotide sequence of SEQ ID NO. 162.

6. A recombinant expression vector comprising the DNA molecule as claimed in claim 4 or claim 5 operably linked to a transcription regulatory element.

7. An isolated cell comprising the recombinant expression vector as claimed in claim 6.

8. A method for producing a *P.gingivalis* polypeptide comprising culturing the cell as claimed in claim 7 under conditions that permit expression of the polypeptide.

9. A pharmaceutical composition comprising:
(i) an effective amount of (a) at least one polypeptide as claimed in claim 1, 2 or 3, (b) at least one DNA molecule as claimed in claim 4 or claim 5, and/or (c) a polypeptide of at least 40 amino acids having a contiguous sequence of at least 40 amino acids identical to a contiguous amino acid sequence of SEQ ID NO. 426, wherein the polypeptide is capable of raising an immune response against *P*. *gingivalis;* and
(ii) a pharmaceutically acceptable carrier.

10. A composition as claimed in claim 9 or claim 10 in which the pharmaceutically acceptable carrier is an adjuvant.

11. A composition as claimed in claim 9 or claim 10 for use in raising an immune response directed to *P.gingivalis* in a subject.

12. A composition for use as claimed in claim 11 for treating a subject for *P.gingivalis* infection.

13. A composition for use as claimed in claim 12, wherein the treatment is a prophylactic treatment or a therapeutic treatment.

14. An antibody raised against a polypeptide as claimed in claim 1, 2 or 3.

15. An antibody as claimed in claim 14 in which the antibody is polyclonal or monoclonal.

16. A composition comprising at least one antibody as claimed in claim 14 or claim 15.

17. A composition as claimed in claim 16 which is adapted for oral use.

18. A method for detecting the presence of a DNA molecule as claimed in claim 4 in a sample comprising:
(a) contacting a sample with the nucleotide probe comprising at least 18 nucleotides and having a contiguous sequence of at least 18 nucleotides identical to a contiguous nucleotide sequence of SEQ ID NO. 37 under conditions in which a hybrid can form between the probe and the DNA molecule as claimed in claim 4 in the sample; and
(b) detecting the hybrid formed in step (a), wherein detection of a hybrid indicates the presence of the DNA molecule as claimed in claim 4 in the sample.

## Patentansprüche

1. Isoliertes antigenes *Porphyromonas gingivalis-*Polypeptid, wobei das Polypeptid
(i) die Aminosäuresequenz von SEQ ID NO. 426,
(ii) die Aminosäuresequenz der Reste 24 bis 821 oder 27 bis 821 von SEQ ID NO. 426 oder
(iii) eine Aminosäuresequenz, die zu mindestens 85 % identisch mit der Aminosäuresequenz von (i) ist, umfasst.

2. Polypeptid nach Anspruch 1, das eine Aminosäuresequenz umfasst, die zu mindestens 95 % identisch mit der Aminosäuresequenz von (i) ist.

3. Polypeptid nach Anspruch 1 oder Anspruch 2, wobei die Aminosäuresequenz SEQ ID NO. 426 ist.

4. Isoliertes DNA-Molekül, wobei das DNA-Molekül eine Nucleotidsequenz umfasst, die für das Polypeptid nach Anspruch 1, 2 oder 3 codiert.

5. Isoliertes DNA-Molekül nach Anspruch 4, wobei das DNA-Molekül die Nucleotidsequenz SEQ ID NO. 162 umfasst.

6. Rekombinanter Expressionsvektor, der das DNA-Molekül nach Anspruch 4 oder Anspruch 5 funktional verknüpft mit einem Transkriptionsregulationselement umfasst.

7. Isolierte Zelle, die den rekombinanten Expressionsvektor nach Anspruch 6 umfasst.

8. Verfahren zur Herstellung eines *P*. *gingivalis-*Polypeptids, wobei das Verfahren das Kultivieren der Zelle nach Anspruch 7 unter Bedingungen, die eine Expression des Polypeptids ermöglichen, umfasst.

9. Pharmazeutische Zusammensetzung, die umfasst:
(i) eine wirksame Menge von (a) mindestens einem Polypeptid nach Anspruch 1, 2 oder 3, (b) mindestens einem DNA-Molekül nach Anspruch 4 oder 5 und/oder (c) einem Polypeptid von mindestens 40 Aminosäuren mit einer fortlaufenden Sequenz von mindestens 40 Aminosäuren, die identisch mit einer fortlaufenden Aminosäuresequenz von SEQ ID NO. 426 ist, wobei das Polypeptid eine Immunreaktion gegen *P. gingivalis* bewirken kann; und
(ii) einen pharmazeutisch akzeptablen Träger.

10. Zusammensetzung nach Anspruch 9, wobei der pharmazeutisch akzeptable Träger ein Adjuvans ist.

11. Zusammensetzung nach Anspruch 9 oder Anspruch 10 zur Verwendung zum Bewirken einer auf *P*. *gingivalis* gerichteten Immunreaktion in einem Subjekt.

12. Zusammensetzung zur Verwendung nach Anspruch 11 zur Behandlung eines Subjekts in Bezug auf eine *P. gingivalis-*Infektion.

13. Zusammensetzung zur Verwendung nach Anspruch 12, wobei die Behandlung eine prophylaktische Behandlung oder eine therapeutische Behandlung ist.

14. Antikörper, der gegen ein Polypeptid nach Anspruch 1, 2 oder 3 gebildet ist.

15. Antikörper nach Anspruch 14, wobei der Antikörper polyklonal oder monoklonal ist.

16. Zusammensetzung, die mindestens einen Antikörper nach Anspruch 14 oder Anspruch 15 umfasst.

17. Zusammensetzung nach Anspruch 16, die zur oralen Verwendung angepasst ist.

18. Verfahren zur Detektion des Vorhandenseins eines DNA-Moleküls nach Anspruch 4 in einer Probe, umfassend:
(a) Inkontaktbringen einer Probe mit der Nucleotidsonde, die mindestens 18 Nucleotide umfasst und eine fortlaufende Sequenz von mindestens 18 Nucleotiden aufweist, die mit einer fortlaufenden Nucleotidsequenz von SEQ ID NO. 37 identisch ist, unter Bedingungen, unter denen ein Hybrid zwischen der Sonde und dem DNA-Molekül nach Anspruch 4 in der Probe gebildet werden kann; und
(b) Detektieren des in Stufe (a) gebildeten Hybrids, wobei die Detektion eines Hybrids das Vorhandensein des DNA-Moleküls nach Anspruch 4 in der Probe anzeigt.

## Revendications

1. Polypeptide de *Porphyromonas gingivalis* antigénique isolé, le polypeptide comprenant :
(i) la séquence d'acides aminés de SEQ ID NO : 426 ;
(ii) la séquence d'acides aminés des résidus 24 à 821 ou 27 à 821 de SEQ ID NO : 426 ; ou
(iii) une séquence d'acides aminés au moins à 85 % identique à une séquence d'acides aminés de (i).

2. Polypeptide selon la revendication 1, comprenant une séquence d'acides aminés au moins à 95 % identique à une séquence d'acides aminés de (i).

3. Polypeptide selon la revendication 1 ou la revendication 2, où la séquence d'acides aminés est la SEQ ID NO : 426.

4. Molécule d'ADN isolée, la molécule d'ADN comprenant une séquence nucléotidique qui code pour le polypeptide selon la revendication 1, 2 ou 3.

5. Molécule d'ADN isolée selon la revendication 4 dans laquelle la molécule d'ADN comprend la séquence nucléotidique de SEQ ID NO : 162.

6. Vecteur d'expression recombinant comprenant la molécule d'ADN selon la revendication 4 ou la revendication 5 liée de manière fonctionnelle à un élément de régulation transcriptionnelle.

7. Cellule isolée comprenant le vecteur d'expression recombinant selon la revendication 6.

8. Procédé de production d'un polypeptide de *P. gingivalis* consistant à mettre en culture la cellule selon la revendication 7 dans des conditions qui permettent l'expression du polypeptide.

9. Composition pharmaceutique comprenant :
(i) une quantité efficace de (a) au moins un polypeptide selon la revendication 1, 2 ou 3, (b) au moins une molécule d'ADN selon la revendication 4 ou la revendication 5, et/ou (c) un polypeptide d'au moins 40 acides aminés ayant une séquence contiguë d'au moins 40 acides aminés identique à une séquence d'acides aminés contigüe de SEQ ID NO : 426, où le polypeptide est capable de provoquer une réponse immunitaire contre *P. gingivalis* ; et
(ii) un support pharmaceutiquement acceptable.

10. Composition selon la revendication 9 ou la revendication 10 dans laquelle le support pharmaceutiquement acceptable est un adjuvant.

11. Composition selon la revendication 9 ou la revendication 10 destinée à être utilisée pour provoquer une réponse immunitaire dirigée contre *P. gingivalis* chez un sujet.

12. Composition destinée à une utilisation selon la revendication 11 pour traiter un sujet atteint d'une infection due à *P. gingivalis.*

13. Composition destinée à une utilisation selon la revendication 12, où le traitement est un traitement prophylactique ou un traitement thérapeutique.

14. Anticorps mobilisé contre un polypeptide selon la revendication 1, 2 ou 3.

15. Anticorps selon la revendication 14, lequel anticorps est polyclonal ou monoclonal.

16. Composition comprenant au moins un anticorps selon la revendication 14 ou la revendication 15.

17. Composition selon la revendication 16 qui est adaptée pour une utilisation par voie orale.

18. Procédé pour détecter la présence d'une molécule d'ADN selon la revendication 4 dans un échantillon consistant à :
(a) mettre en contact un échantillon avec la sonde nucléotidique comprenant au moins 18 nucléotides et ayant une séquence contiguë d'au moins 18 nucléotides identique à une séquence nucléotidique contiguë de SEQ ID NO : 37 dans des conditions dans lesquelles un hybride peut se former entre la sonde et la molécule d'ADN selon la revendication 4 dans l'échantillon ; et
(b) détecter l'hybride formé dans l'étape (a), où la détection d'un hybride indique la présence de la molécule d'ADN selon la revendication 4 dans l'échantillon.
